# EUROPEAN PATENT SPECIFICATION

(11) **EP 1 525 219 B1**
(45) Date of publication and mention of the grant of the patent: **27.05.2009**
(21) Application number: 03762471.5
(22) Date of filing: 02.07.2003
(51) Int. Cl.: C07K 14/575, C07K 14/605, A61K 38/26, A61P 3/10

(54) **GLP-1 AND METHODS FOR TREATING DIABETES**
GLP-1 UND BEHANDLUNGSMETHODE FÜR DIABETES
GLP-1 ET METHODES DE TRAITEMENT DU DIABETE

(30) Priority: 04.07.2002 US 393917 P; 24.04.2003 US 465613 P
(43) Date of publication of application: 27.04.2005
(60) Divisional application: 08016668.9
(73) Proprietor: Zealand Pharma A/S, 2600 Glostrup (DK)
(72) Inventor: STEINESS, Eva, DK-2900 Hellerup (DK)
(74) Representative: Kiddle, Simon John
(86) International application number: PCT/DK2003/000463
(87) International publication number: WO 2004/005342

(56) References cited:
- EP-A- 1 329 458
- WO-A-01/04156
- WO-A-01/32158
- WO-A-99/43708
- US-B1- 6 344 180
- US-B1- 6 358 924
- ANDREW A. YOUNG ET AL: "Studies in Obese Diabetic (ob/ob, db/db) Mice, Diabetic Fatty Zucker Rats, and Diabetic Rhesus Monkeys (Macaca mulatta)" DIABETES, vol. 48, May 1999 (1999-05), pages 1026-1034, XP002256109
- LISA JUNTTI-BERGGREN ET AL: "The Antidiabetogenic Effect of GLP-1 Is Maintained During a 7-Day Treatment Period and Improves Diabetic Dyslipoproteinemia in NIDDM Patients" DIABETES CARE, vol. 19, no. 11, November 1996 (1996-11), pages 1200-1206, XP002256110
- DAVID G. PARKES ET AL: "Insulinotropic Actions of Exendin-4 and Glucagon-Like Peptide-1 In Vivo and In Vitro " METABOLISM, vol. 50, no. 5, May 2001 (2001-05), pages 583-589, XP002256111
- RÉMY BURCELIN ET AL: "Long-Lasting Antidiabetic Effect of a Dipeptidyl Peptidase IV-Resistant Analog of Glucagon-Like Peptide-1" METABOLISM, vol. 48, no. 2, February 1999 (1999-02), pages 252-258, XP002256112
- U RITZEL ET AL: "A synthetic glucagon-like peptide-1 analog with improved plasma stability" JOURNAL OF ENDOCRINOLOGY, vol. 159, 1998, pages 93-102, XP002256113
- DORIS A. STOFFERS ET AL: "Insulinotropic Glucagon-Like Peptide 1 Agonists Stimulate Expression of Homeodomain Protein IDX-1 and Increase Islet Size in Mouse Pancreas " DIABETES, vol. 49, May 2000 (2000-05), pages 741-748, XP002256114

## Description

### FIELD OF THE INVENTION

The present invention generally relates to medical uses for treating diabetes and related disorders in a mammal. In one aspect, the uses involve administering glucagon like peptide (GLP-1) or a GLP-1 against having GLP-1 like effect to provide a therapeutically effective amount of endogenous insulin. Subsequently, the amount of drug administered to the mammal is substantially reduced to produce a "drug holiday". Practice of the invention achieves effective therapy without continuous drug exposure and without continuous presence of therapeutic levels of the drug.

### BACKGROUND

Glucagon-like peptide 1 (GLP-1) has been described as a physiological incretin hormone. It reportedly serves to augment an insulin response after an oral intake of glucose or fat. It is generally understood that GLP-1 lowers glucagon concentrations, slows gastric emptying, stimulates (pro)insulin biosynthesis, enhances insulin sensitivity and stimulates the insulin independent glycogen synthesis. See Holst, JJ (1999) Curr Med Chem. 6:1005; Nauck, MA, et al. (1997) Exp Clin Endocrinol Diabetes 105:187; and Lopez-Delgado,MI, et al. (1998) Endocrinology 139:2811.

The molecular structure and function of GLP-1 has been extensively studied. GLP-1 amino acid and protein sequence has been reported for human and other mammals. Human GLP-1 is thought to have a 37 amino acid residue protein. See eg., Heinrich, G., et al., Endocrinol., 115: 2176 (1984); and Uttenthal, L. O., et al., J. Clin. Endocrinol. Metabol., 61:472 (1985).

Many molecular derivatives of GLP-1 have been reported. For instance, GLP-1 (7-37) is know. A variety of analogs have also been described eg., Gln⁹ -GLP-1 (7-37), D-Gln⁹ -GLP-1 (7-37), acetyl-Lys⁹ -GLP-1 (7-37), Thr¹⁶-Lys¹⁸ -GLP-1(7-37), and Lys¹⁸ -GLP-1 (7-37), Gly⁸-GLP-1 (7-37), Ser⁸-GLP-1 (7-37),. Other GLP-1 derivatives (sometimes called "variants") have also been reported particularly as acid addition salts, carboxylate salts, lower alkyl esters, and amides. See WO 91/11457 and Mojsov, S., Int. J. Peptide Protein Research, 40:333-343 (1992), and references cited therein.

Additional GLP-1 derivatives have been disclosed some of which are reported to have agonist activity. See eg., U.S. Patent Nos. 6,358,924; 6,344,180; 6,284,725; 6,277,819; 6,271,241; 6,268,343; and 6,191,102.

Additional GLP-1 related molecules have been disclosed.

For instance, a protein called exendin- 4 has been isolated from the salivary gland of the Gila monster. Further work has shown that the peptide is related to GLP-1. There is recognition that the peptide is a potent agonist for the mammalian GLP-1 receptor. Recent studies have shown that administration of exendin-4 induces pancreatic endocrine differentiation, islet proliferation and an increase in β-cell mass indicating that exendin-4 may exert tropic effects on the β-cells. See Raufman, JP, et al. J (1992) J Biol Chem 267:21432; Young, AA, et al. (1999) Diabetes 48:1026-1034; Edvell,A, Lindström,P (1999) Endocrinology 140:778-783; Xu,G, et al. Diabetes 48:2270; Greig, NH, et al. (1999) Diabetologia 42:45; and Parkes, DG, et al. (2001) Metabolism 50:583.

A variety of molecules related to exendin-4 and exendin-3 have been reported including analogs and derivatives. See eg., U.S. Patent No. 5,424,286; WO98/05351; WO98/30231; and published EP Application No. 99610043.4.

For instance, Larsen, B.D et al. have disclosed novel peptide agonists of GLP-1 activity. See PCT/DK00/00393.

Type II diabetes has been the subject of much investigation. The disease has been characterized by impaired glucose tolerance, hyperinsulaemia, insulin resistance, increases in glycosylated hemoglobin (HbA_{1c}), β-cell dysfunction and subsequent β-cell death. See generally S. N. Davis and D. K. Granner in Goodman & Gilman's The Pharmacological Basis of Therapeutics (9th Ed. Hardman, J.G et al. (eds) (1996)) Chapter 60, pp. 1493-1597.

In particular, infusion of GLP-1 has been reported to normalize the level of HbA_{1c} There are reports that GLP-1 is further capable of enhancing the ability of β-cells to sense and respond to glucose in subjects with impaired glucose tolerance. See Byrne et al., *supra.*

There is general agreement that the db/db mouse is a satisfactory model of human type II diabetes. This diabetic mouse has been characterized by leptin receptor mutation leading to severe obesity, early onset of type II diabetes, hyperinsulinaemia and marked peripheral insulin resistance. Ultimately these animals develop β-cell exhaust and full insulin dependence. Thus, the clinical progression from type II to type I diabetes in *db*/*db* mice is believed to be bear relationship to human type II diabetes. See Coleman, DL (1973) Diabetologia 9:294; and Leiter, EH, et al. (1983) J.Nutr. 113:184.

Despite attempts to understand and address diabetes, there is still no cure for the disease. Reported treatment methods generally involve administration of insulin alone or in conjunction with one or more agents including oral hypoglycemic drugs. See S. N. Davis and D. Granner, *supra.*

Unfortunately, prior methods for preventing and treating diabetes have been problematic.

For instance, insulin therapy has been linked to several adverse reactions eg., hypoglycemia, insulin allergy and resistance, and insulin related edema. In some medical settings, these reactions can be life threatening. Contraindications and side effects abound for most conventional anti-diabetic drugs.

In addition, conventional methods for treating diabetes typically involve multidose regimens in which insulin is administered subcutaneously. The inconvenience, pain and cost associated with such methods can be considerable. Management of diabetic children and the elderly with these highly invasive and repetitive therapies can be especially difficult.

It would be useful to have methods that prevent or treat diabetes and related disorders that generally require less administration of an anti-diabetic formulation. It would be especially useful to have methods that provide a physiologically relevant amount of endogenous insulin, thereby helping to delay need to administer the anti-diabetic formulation to prevent or treat the diabetes and related disorders.

### SUMMARY OF THE INVENTION

The invention generally relates to the treatment and prevention of diabetes and related disorders in a mammal. In one aspect, the invention involves the use of at least one GLP-1 or a GLP-1 agonist having GLP-1 effect for the manufacture of a medicament for preventing or treating diabetes in a mammal, wherein the amount and timing of administration of said medicament are such as to prevent or treat diabetes in the mammal, comprising reducing administration of the GLP-1 or related molecule below about the therapeutically effective amount for a time conducive to producing a drug holiday, wherein the drug holiday is further defined as a time interval between a first endpoint following the reduction in administering the GLP-1 or related molecule and a second endpoint.

In a further aspect, the present invention relates to a GLP-1 or a GLP-1 agonist as recited in claim 2.

It has been discovered that it is possible to prevent or treat diabetes and related disorders by administering GLP-1 and related molecules (drugs) in a new way. More specifically, it has been found that it is not necessary to expose mammals to these drugs continuously to achieve a desired therapeutic effect. That is, it has been found that it is possible to reduce administration of the drug, sometimes substantially, over a time period referred to herein as a "drug holiday". During the drug holiday, the mammal is surprisingly able to provide itself with a useful amount of endogenous insulin. It is believed that the amount of endogenous insulin is generally sufficient to help prevent, treat, delay onset of, reduce symptoms of or delay progression of diabetes and related disorders. Administration of GLP-1 or GLP-1 agonists is not needed over this time period although after the drug holiday, drug administration can be resumed.

Practice of the invention provides important advantages.

For example, I have found that the drug holiday can provide mammals and especially human patients with much sought after relief from invasive, sometimes painful, and often repetitive and expensive therapies. Potentially serious side-effects and related complications often associated with such therapies can be reduced, delayed, or in some instances eliminated by my invention. In particular, risk of developing hypoglycemia, allergy and resistance, and edema and related insulin side effects can be sometimes be reduced or avoided by providing for at least one drug holiday in accord with this invention.

Additionally, costs associated with repeated and frequent dosing of anti-diabetic drugs (eg., GLP-1, GLP-1 agonist, insulin) can be reduced substantially by the methods described herein.

Specific benefits accrue to human patients that have or are suspected of having diabetes or a related disorder including Type II diabetes. For example, the GLP-1 or GLP-1 agonist can be administered to the patient regularly and for a time sufficient to at least maintain and, in some instances, increase endogenous insulin levels. In this embodiment, further administration of the GLP-1 or GLP-1 agonist can be decreased, sometimes substantially, to provide for the drug holiday. During this time, and without wishing to be bound to theory, it is believed that the patient is able to provide him/herself with an amount of endogenous insulin that is therapeutically relevant. That is, the amount of endogenous insulin provided is able to help the patient exert more control over undesired blood glucose fluctuations. Thus; the need to continue or even increase exposure to GLP-1 or GLP-1 agonists can be avoided during the drug holiday by using my invention.

It is a further object of the invention to provide a method to revent or treat diabetes (including related disorders) in which administration of GLP-1 or a GLP-1 agonist having GLP-1 like effect is reduced during the drug holiday period. In one embodiment, administration of the drug is eliminated entirely during the drug holiday period. After or sometimes during the drug holiday period, the GLP-1 or GLP-1 agonist is administered again to the mammal in an amount that is the substantially the same or different from the amount administered previously. That second drug administration can be followed by another drug holiday if desired. Thus it is a feature of the invention to provide for at least one (ie. multiple) drug holidays in which each drug holiday is preferably followed by administration of an amount of at least one of GLP-1, a GLP-1 agonist, or another drug such as those recognized anti-diabetic agents disclosed herein such as insulin and analogues thereof.

Thus in one embodiment, the invention can be used to provide a mammal such as a human patient with a first drug holiday, after which time the GLP-1 or GLP-1 agonist having a GLP-1 like effect is administered to the patient in an amount that is preferably therapeutically effective. This can be repeated once, twice, thrice or as often as needed to provide a therapeutic regimen that features one, two, three or more drug holidays. The invention can be repeated as needed eg., every day, every few days, every few weeks, every few months up to the lifetime of the mammal to prevent or treat a medical condition. Human patients who are known to have diabetes or who are suspected to be susceptible to same (eg., those with or suspected of having impaired glucose tolerance (IGT)) are expected to benefit particularly from the invention.

Additionally, the present invention may include reducing administration of the GLP-1 or GLP-1 agonist below about the therapeutically effective during the drug holiday. Additional features of the drug holiday are discussed below.

Prior to induction of the drug holiday, the amount of GLP-1 or GLP-1 agonist administered to the mammal is preferably, but not exclusively, one that is therapeutically effective. In one embodiment and without wishing to be bound to theory, the amount is sufficient to help the mammal maintain or even increase endogenous insulin levels (before or during the drug holiday). To begin the drug holiday, the amount of administered drug is reduced or eliminated entirely. The drug holiday period is not tied to any particular endogenous insulin level(s) or method of providing or producing that insulin *in vivo* so long as that during the drug holiday, the mammal is able to provide itself with some amount of useful insulin, preferably an amount that is therapeutically relevant and effective. It has been found that presence of such endogenous insulin during the drug holiday period is exceptionally useful for treating or preventing diabetes and related disorders in the mammal. Following the drug holiday period, the mammal can be subjected to additional therapy including further administration of at least one of GLP-1, a GLP-1 agonist , and recognized anti-diabetic agents such as those mentioned herein.

Other features of the invention are described below.

### BRIEF DESCRIPTION OF THE DRAWINGS

Figure 1 is graph showing plasma insulin levels following administration of vehicle (control) and 100nmol/kg of Compound 1.
Figure 2 is a graph showing blood glucose levels (nM) after overnight fasting (17 hours)
Figure 3 is a graph showing results of an oral glucose tolerance test (OTT). Results are expressed as AUC_{0-240 min} (mM x min) per day.
Figure 4 is showing amounts of glycosylated hemoglobin (HbA_{1c}) in groups exposed to vehicle or varying concentration of Compound 1.
Figure 5 is a graph showing fasting blood glucose levels following treatment with vehicle or various Compound 1 administration strategies.
Figure 6 is a graph illustrating results from an oral glucose tolerance test (OGTT) after vehicle treatment or various Compound 1 administration strategies. Results are expressed as dAUC_{0-240 min} (mM x min).
Figure 7 is a graph showing pancreatic insulin mRNA levels (pg/microgram total RNA) after treatment with vehicle or various Compound 1 administration strategies.
Figure 8 is a graph showing amounts of HbA_{1c} (% of total haemoglobin) in groups exposed to vehicle or various Compound 1 administration routes.

### DETAILED DESCRIPTION OF PREFERRED EMBODIMENTS

As discussed, the invention features medical uses for preventing or treating diabetes in a mammal. Particular mammals that would be expected to benefit from practice of the invention include those that have, have had, are suspected of having, or are genetically or otherwise disposed to develop diabetes or a related medical condition. Particular mammals of interest include primates such as chimpanzees; rodents such as mice and rats; domesticated animals eg., rabbits, dogs, pigs, cats, sheep, goats, horses, cows and the like. More specific mammals of interest include accepted animal models of diabetes and related disorders such as the mouse model described below. An especially preferred mammal for use with the invention is a human patient who has been diagnosed with diabetes or a related disease such as impaired glucose tolerance (IGT).

As also discussed, the present invention provides medical uses for treating or preventing diabetes in a mammal, including related disorders, which includes administering to the mammal a therapeutically effective amount of at least one of GLP-1 and a GLP-1 agonist having GLP-1 effect. Typically, the amount and timing of administration are such as to prevent or treat diabetes in the mammal without the continuous presence of the molecule. Preferably such a method further includes reducing administration of the GLP-1 or GLP-1 agonist below about the therapeutically effective amount for a time conducive to producing a drug holiday. Preferred methods are generally sufficient to prevent or treat the diabetes or related disorder in the mammal.

By the phrase "therapeutically relevant amount of endogenous insulin" is meant an insulin amount provided by the mammal that is at least sufficient to at least slow progression of diabetes or a related disorder in that mammal by at least about 10%, preferably at least 25% or at least about 50%. Progression of such disorders can be determined by one or a combination of recognized clinical tests such as the ability to utilize glucose or the level of glycosylated hemoglobin in the bloodstream. Preferred tests are described in more detail below. Illustrative amounts of endogenous insulin that are known in the field to be therapeutically relevant are provided below. However, it is emphasized that the invention is not tied to any particular level, amount, or production of endogenous insulin. It is recognized that the ability of a subject mammal to provide itself with at least some endogenous insulin can be manifested in an improved ability to utilize glucose eg., as determined by the tests described herein.

In one embodiment of the present invention, and typically prior to the start of the drug holiday, administration of at least one of GLP-1 or GLP-1 agonist is reduced during the drug holiday by at least about 50% below the therapeutic amount, preferably at least about 90% below the therapeutic amount, and more preferably such administration is stopped during the drug holiday.

By the phrase "GLP-1 related molecule" is meant a derivative, homologue, variant or analog of GLP-1 including pharmaceutically acceptable salts and free acids thereof. Preferably, the GLP-1 related molecule is a GLP agonist. More specific GLP-1 and GLP-1 related molecules are provided below. GLP-1 analogs, derivatives, variants, precursors and homologues are all suitable for the practice of the invention as long as the active fragment that impacts endogenous insulin production is included. By "GLP-1" is meant GLP-1(7-37). By custom, the amino-terminus of GLP-1(7-37) has been assigned number 7 and the carboxy-terminus, number 37.

The amino acid sequence of GLP-1(7-37) is well-known and has the following sequence: NH₂-His⁷ -Ala-Glu-Gly¹⁰ -Thr-Phe-Thr-Ser-Asp¹⁵ -Val-Ser-Ser-Tyr-Leu²⁰ -Glu-Gly-Gln-Ala-Ala²⁰ -Lys-Glu-Phe-Ile-Ala³⁰ -Trp-Leu-Val-Lys-Gly-Arg-Gly³⁷ -COOH.

A "GLP-1 analog" is defined as a molecule having a modification including one or more amino acid substitutions, deletions, inversions, or additions when compared with GLP-1. GLP-1 analogs include, for example, GLP-1(7-34) and GLP-1(7-35), GLP-1(7-36), Val⁸-GLP-1(7-37), Gln⁹ -GLP-1(7-37), D-Gln⁹ -GLP-1(7-37), Thr¹⁶ -Lys¹⁸ -GLP-1(7-37), and Lys¹⁸ -GLP-1(7-37). Preferred GLP-1 analogs are GLP-1(7-34) and GLP-1(7-35), which are disclosed in U.S. Pat. No. 5,118,666, and also GLP-1(7-36). These compounds are the biologically processed forms of GLP-1 having insulinotropic properties. Other GLP-1 analogs are disclosed in U.S. Pat. No. 5,545,618.

By the phrase "GLP-1 derivative" is meant a molecule having the amino acid sequence of GLP-1 or of a GLP-1 analog, but additionally having at least one chemical modification of one or more of its amino acid side groups, alpha-carbon atoms, terminal amino group, or terminal carboxylic acid group. A chemical modification includes adding chemical moieties, creating new bonds, and removing chemical moieties. Modifications at amino acid side groups include acylation of lysine epsilon-amino groups, N-alkylation of arginine, histidine, or lysine, alkylation of glutamic or aspartic carboxylic acid groups, and deamidation of glutamine or asparagine. Modifications of the terminal amino include the desamino, N-lower alkyl, N-di-lower alkyl, and N-acyl modifications. Modifications of the terminal carboxy group include the amide, lower alkyl amide, dialkyl amide, and lower alkyl ester modifications. A lower alkyl is a C₁ -C₄ alkyl. Furthermore, one or more side groups, or terminal groups, may be protected by protective groups known to the ordinarily-skilled protein chemist. The α-carbon of an amino acid may be mono- or di-methylated.

As discussed, the invention is compatible with use of a wide spectrum of GLP-1 analogs and derivatives. Further examples include active GLP-1 peptides, 7-34, 7-35, 7-36 and 7-37 have amino acid substitutions as positions 7-10 and/or are truncated at the C-terminus and/or contain various other amino acid substitutions in the basic peptide. Analogs having D-amino acid substitutions in the 7 and 8 positions and/or N-alkylated or N-acylated amino acids in the 7 position are particularly resistant to degradation in vivo.

See also U.S Pat. Nos. 6,358,924; 6,344,180; 6,284,725; 6,277,819; 6,271,241; 6,268,343; 6,191,102; 6,051,689; 6,006,753; 5,846,937; 5,670,360; 5,614,492; 5,846,937; 5,545,618; 6,410,508; 6,388,053; 6,384,016; 6,329,336; 6,110,703, 5,846,747; 5,670,360; and 5,631,224 (disclosing additional GLP-1 and related molecules) .

As mentioned, it is an invention object to provide subject mammals with at least one drug holiday eg., one, two, three or more of such drug holidays. It will be appreciated that the length of time associated with any particular drug holiday will vary depending on recognized parameters such as the health of the subject, sex, disease to be treated, weight, etc.

A preferred drug holiday is defined as the time interval between a first endpoint (start) and a second endpoint (finish). Typically, the first endpoint follows the reduction in administration of the GLP-1 or related molecule. The second endpoint can be readily identified by one or a combination of standard methods. For example, such endpoints can be characterized by an inability of the subject to control fasting blood glucose (FBG) as demonstrated, for instance, by an increase in FBG of at least about 5% or 10% when compared to the time prior to the second endpoint. The second endpoint can be further identified by an unwanted increase in glycosylated hemoglobin of at least about 5% or 10%, also when compared to the interval before the second endpoint. Methods for determining FBG and glycosylated hemoglobin are known and include what is referred to herein as a standard FBG or glycosylated hemoglobin test, respectively. During a preferred drug holiday period, FBG and glycosylated hemoglobin do not increase significantly.

As stated, the length of time associated with a particular drug holiday will vary depending on recognized factors including the health of the subject, sex, disease to be treated, weight, medical history, etc. In one invention embodiment however, the drug holiday spans about one day up to about twenty five weeks eg., between from about three to four weeks. However, as mentioned above, at or near the end of a drug holiday period, the standard FBG assay will show an increase in FBG of at least about 5% or 10%.

The GLP-1 or related molecule administered in accord with this invention can be given by nearly any acceptable route including a depot formulation such as those described in US Patent Nos. 6,358,924; 6,344,180; 6,284,725; 6,277,819; 6,271,241; 6,268,343; 6,191,102; 6,051,689; 6,006,753; 5,846,937; 5,670,360; 5,614,492; 5,846,937; 5,545,618; 6,410,508; 6,388,053; 6,384,016; 6,329,336; 6,110,703, 5,846,747; 5,670,360; and 5,631,224. Alternatively, or in addition, such drugs can be administered to the mammal bolus at least about once daily, at least once a week. Other administration routes are also envisioned including about twice daily (i.v. or subq) for between from about one to about twenty weeks.

As mentioned, it is an object of the present invention to provide at least one drug holiday eg., one, two, or three of same to prevent or treat diabetes or a related disorder. The precise number of drug holidays needed to practice the method will depend on recognized parameters such as the health of the subject, sex, disease to be treated, etc. In one embodiment, the invention further includes administering to the mammal a second therapeutically effective amount of GLP-1 or a GLP-1 agonist following the (first) drug holiday. If desired, the method can also include reducing administration of the second therapeutically effective amount of GLP-1 or GLP-1 agonist for a time conducive to producing a second drug holiday. Such administration and reducing steps are repeated at least once eg., at least about 2 to about 25 times or as needed to prevent or treat the diabetes or related disorder. For instance, the invention can be practiced over the lifetime of the mammal.

As also discussed, the invention also features methods which include administering to the mammal a therapeutically effective amount of at least one of glucagon-like peptide 1 (GLP-1), or a GLP-1 agonist to provide a therapeutically effective amount of endogenous insulin in the mammal. It is an invention objective to provide a respite (drug holiday) from conventional anti-diabetes therapies by helping to at least maintain and preferably increase endogenous insulin production in the mammal. An invention goal is to at least delay resumption of such therapy during the drug holiday period.

General methods for characterizing, diagnosing and treating human diabetes have been disclosed by S. N. Davis and D. Granner, *supra*; as well as references cited therein.

In one embodiment of the present invention method, the endogenous insulin production is at least about maintained in the mammal for between from about one day to about twenty five weeks. In another embodiment, administration of the GLP-1, GLP-1 agonist is sufficient to increase the insulin production in the mammal by at least about 10% compared to a control. Preferably, the increase in endogenous insulin production is at least about 20%, more preferably at least about a 50% increase compared to the control. A preferred control is a diabetic, non-treated mammal.

Reference herein to a control mammal typically means untreated with the GLP-1 or GLP-1 agonist. Suitable controls are mammals that may be diabetic or non-diabetic as needed to suit a particular invention use. It will be appreciated that for some invention applications, use of a control will not be needed eg., as when endogenous insulin production levels are already known.

Methods for detecting and measuring insulin levels and insulin mRNA (protein and nucleic acid levels) are routine. See generally S. N. Davis and D. Granner, *supra*; as well as references cited therein. Thus in one example of the invention, the method further includes monitoring at least one of endogenous insulin production and blood glucose levels. Such monitoring can, for instance, include detecting insulin mRNA in the mammal. In this embodiment, the mammal will be a non-human mammal to allow for collection and analysis of insulin producing tissue, usually a pancreatic biopsy. In human patients, less invasive monitoring of blood insulin production according to establish protocols will be helpful for some invention applications. Methods for at least detecting insulin mRNA from biological samples are known and include nucleic acid hybridization, PCR and related amplification techniques. Thus in an example of the invention, the method further includes quantitating the insulin mRNA and comparing the amount produced by the mammal to a control. Acceptable plasma insulin tests are described below.

Therapeutically relevant levels of insulin are known for a wide variety of mammals. For human patients, it has been reported that endogenous insulin circulates in the blood as a monomer at a concentration of between from about 2 to about 4 ng/ml in the portal blood and in the peripheral circulation at about 0.5ng/ml or about 0.1mn. After ingestion of a meal, there is believed to be a rise in the concentration of endogenous insulin in the portal blood, followed by a smaller rise in the periphery. See S. N. Davis and D. Granner, *supra.*

As discussed, the invention is not tied to attaining the aforementioned levels of insulin which have been reported to be therapuetically important. Rather, there is general recognition in the field that any amount of insulin provided endogenously is relevant in most therapuetic settings. That is, it is generally preferred that a subject mammal have capacity to provide some amount of endogenous insulin regardless of whether it achieves normal or near normal levels. Even presence of some endogenous insulin as provided by this invention will help at least slow disease progression and in some instances make it more clinically manageable.

A "therapeutically effective" amount of GLP-1 or GLP-1 agonist administered in accord with this invention means restoring at least about 0.01% of the normal monomeric concentration of insulin (eg. human insulin) in the portal blood and peripheral circulation, preferably at least about 0.5% of same, more preferably at least about 5%, and even more preferably at least about 10% of those levels. Additionally preferred dosages of the GLP-1 and GLP-1 agonist will further approach endogenous levels of insulin production in subject mammals eg., by at least about 50% up to 100% to 200% of same. More particular examples of such amounts can be found in one or more of the following patents: 6,358,924; 6,344,180; 6,284,725; 6,277,819; 6,271,241; 6,268,343; 6,191,102; 6,051,689; 6,006,753; 5,846,937; 5,670,360; 5,614,492; 5,846,937; 5,545,618; 6,410,508; 6,388,053; 6,384,016; 6,329,336; 6,110,703, 5,846,747; 5,670,360; and 5,631,224.

In many embodiments, the GLP-1 or GLP-1 agonist can be administered to the mammal at a dose of at least about 0.01 nmol/kg (body weight).

Thus an example of a drug holiday, in one invention embodiment, will achieve at least about 50% of the levels of insulin in the portal blood and peripheral circulation as in normal (non-diabetic) subjects, preferably at least about 80%, more preferably about 100% of same.

It will be appreciated that the baseline level of insulin production in most subject mammals can be ascertained by workers in the field. Typically normal insulin levels for most healthy human subjects are known and are understood to vary by age, sex, diet and health of particular individuals. For subjects who have or are suspected of having diabetes, that baseline can change but is ascertainable using standard methods.

In one embodiment of the method, the amount of insulin produced by the mammal is at least about 10% higher than a control as determined by a standard plasma insulin test, preferably at least about 20% higher, more preferably at least about 50% higher. A preferred control is a non-treated diabetic mammal. Conventional methods for detecting plasma insulin are disclosed below. See also S. N. Davis and D. Granner, *supra.*

In another embodiment, the administration of the GLP-1 or GLP-1 agonist is at least about once daily for at least about a 24 hours. Preferably, the administration is about twice daily for between from about one to about twenty weeks. Particular amounts of the GLP-1, or GLP-1 agonist to administer to the mammal will vary with intended use but will generally be at least about 0.01 nmol/kg (body weight), preferably at least about 0.1 nmol/kg (body weight), more preferably 1, 2, 5 or 10 nmol/kg (body weight). More particular amounts of GLP-1 or GLP-1 agonist to use will be guided by recognized parameters including the general health of the subject, type of diabetes, sex, medical history, ect.

Use of the present invention is also fully compatible with use of exendin-4, exendin-3, as well as analogs and derivatives thereof including pharmaceutically acceptable salts of those molecules. More particular examples of such molecules have been reported in U.S. Patent No. 5,424,286; WO98/05351; WO98/30231; WO99/07404, WO 99/25727; WO 99/25728; WO 99/46283; WO01/004156; and published EP 1076066,

As disclosed in the WO 01/004516, for instance, a particular GLP-1 analog includes a peptide X selected from the group consisting of:
(a) an exendin having at least 90% homology to exendin-4;
(b) a variant of said exendin wherein said variant comprises a modification selected from the group consisting of between one and five deletions at positions 34-39 and contains a Lys at position 40 having a lipophilic substituent; or
(c) GLP-1 (7-36) or GLP-1 (7-37) having at least one modification selected from the group consisting of:
   (i) substitution of D-alanine, glycine or alpha-amino isobutyric acid for alanine at position 8 and
   (ii) a lipophilic substituent;
   and Z, a peptide sequence of 4-20 amino acid units covalently bound to said variant, wherein each amino acid unit in said peptide sequence, Z is selected from the group consisting of Ala, Leu, Ser, Thr, Tyr, Asn, Gln, Asp, Glu, Lys, Arg, His, Met, Orn, and amino acid units of the general formula I

   -NH-C(R¹)(R²)-C(=O)- (I)

   wherein R¹ and R² are selected from the group consisting of hydrogen, C₁₋₆-alkyl, phenyl, and phenyl-methyl, wherein C₁₋₆-alkyl is optionally substituted with from one to three substituents selected from halogen, hydroxy, amino, cyano, nitro, sulfono, and carboxy, and phenyl and phenyl-methyl is optionally substituted with from one to three substituents selected from C₁₋₆-alkyl, C₂₋₆-alkenyl, halogen, hydroxy, amino, cyano, nitro, sulfono, and carboxy, or R¹ and R² together with the carbon atom to which they are bound form a cyclopentyl, cyclohexyl, or cycloheptyl ring, e.g. 2,4-diaminobutanoic acid and 2,3-diaminopropanoic acid; and a pharmaceutically acceptable salt or the C-terminal amide of said peptide conjugate.

More particular examples of GLP-1 and GLP-1 agonists including analogs thereof such as those disclosed in WO 01/004156. Such molecules include the following specific compounds:
des Ser³⁹-exendin-4(1-39)-Lys₆-NH₂
des Pro³⁶-exendin-4(1-39)-Lys₆-NH₂
des Ala³⁵-exendin-4(1-39)-Lys₆-NH₂
des Gly³⁴-exendin-4(1-39)-Lys₆-NH₂
des Ser³⁹-(Lys⁴⁰ (palmitoyl))exendin-4(1-39)-Lys₇-NH₂
des Gly³⁴-(Lys⁴⁰ (palmitoyl))exendin-4(1-39)-Lys₇-NH₂
des Ala³⁵-(Lys⁴⁰ (palmitoyl))exendin-4(1-39)-Lys₇-NH₂
des Pro³⁶-(Lys⁴⁰ (palmitoyl))exendin-4(1-39)-Lys₇-NH₂
Lys⁴⁰ (palmitoyl)exendin-4(1-39)-Lys₇-NH₂
des Pro³⁶, Pro³⁷-exendin-4(1-39)-Lys₆-NH₂,
Lys₆-des Pro³⁶, Pro³⁷, Pro³⁸-exendin-4(1-39)-NH₂,
Asn(Glu)₅-des Pro³⁶, Pro³⁷, Pro³⁸-exendin-4(1-39)-NH₂,
Lys₆-des Pro¹⁶, Pro³⁷, Pro³⁸-exendin-4(1-39)-Lys₆-NH₂,
Asn(Glu)₅-des Pro³⁶, Pro³⁷, Pro³⁸-exendin-4(1-39)-Lys₆-NH₂,
des Pro³⁶, Pro³⁷, P³⁸-exendin-4(1-39)-Lys₆-NH₂,
Gly⁸-GLP-1(7-36)-Lys₆-NH₂
Lys₆-Gly⁸-GLP-1(7-36)-Lys₆-NH₂,
Lys⁶-Gly⁸-GLP-1(7-36)-NH₂,
(Gly⁸,Lys³⁷(palmitoyl)-GLP-1(7-36)(Human)-Lys₇-NH₂
(Gly⁸,Lys²⁶(palmitoyl)-GLP-1(7-36)(Human)-Lys₆-NH₂,
Gly⁸,Lys³⁴(palmitoyl)-GLP-1(7-36)(Human)-Lys₆-NH₂,
Gly⁸-GLP-1(7-36)-Lys₈-NH₂,
Gly⁸-GLP-1(7-36)-Lys₁₀-NH₂,
Gly⁸-GLP-1(7-37)-Lys₆-NH₂; and the free acid or pharmaceutically acceptable salt thereof.

Another preferred group of active compounds for use in the present invention is disclosed in WO 91/11457 and includes GLP-1(7-34), GLP-1(7-35), GLP-1(7-36), or GLP-1(7-37), or the amide form thereof, and pharmaceutically-acceptable salts thereof, having at least one modification including those shown below:
(a) substitution of glycine, serine, cysteine, threonine, asparagine, glutamine, tyrosine, alanine, valine, isoleucine, leucine, methionine, phenylalanine, arginine, or D-lysine for lysine at position 26 and/or position 34; or substitution of glycine, serine, cysteine, threonine, asparagine, glutamine, tyrosine, alanine, valine, isoleucine, leucine, methionine, phenylalanine, lysine, or a D-arginine for arginine at position 36;
(b) substitution of an oxidation-resistant amino acid for tryptophan at position 31;
(c) substitution of at least one of: tyrosine for valine at position 16; lysine for serine at position 18; aspartic acid for glutamic acid at position 21; serine for glycine at position 22; arginine for glutamine at position 23; arginine for alanine at position 24; and glutamine for lysine at position 26; and
(d) substitution of at least one of: glycine, serine, or cysteine for alanine at position 8; aspartic acid, glycine, serine, cysteine, threonine, asparagine, glutamine, tyrosine, alanine, valine, isoleucine, leucine, methionine, or phenylalanine for glutamic acid at position 9; serine, cysteine, threonine, asparagine, glutamine, tyrosine, alanine, valine, isoleucine, leucine, methionine, or phenylalanine for glycine at position 10; and glutamic acid for aspartic acid at position 15; and
(e) substitution of glycine, serine, cysteine, threonine, asparagine, glutamine, tyrosine, alanine, valine, isoleucine, leucine, methionine, or phenylalanine, or the D- or N-acylated or alkylated form of histidine for histidine at position 7; wherein, in the substitutions is (a), (b), (d), and (e), the substituted amino acids can optionally be in the D-form and the amino acids substituted at position 7 can optionally be in the N-acylated or N-alkylated form.

See also U.S PatNos. 5,512,549; 5,120,712; 5,118,666; 5,120,712 and 5,523,549 for releated disclosure.

As discussed, the present invention provides important medical uses for preventing or treating diabetes. Preferred practice involves at least maintaining endogenous insulin production in the mammal sufficient to support a drug holiday or maintaining the level of glycosylated hemoglobin sufficient to support a drug holiday. During this time, administration of an anti-diabetic formulation can be significantly reduced and sometimes avoided completely. This in one invention embodiment, the uses will further include administering at least one anti-diabetic drug to the mammal. Preferably, the administration will be about below the recognized therapeutically effective amount for at least one of the drugs in the mammal. That is, the amount of at least one and preferably all of the anti-diabetic drugs given to the mammal will be below about what is accepted as the useful dose. However in other embodiments such as when addressing severe or hard to manage diabetes, it will be helpful to administer at least about at a therapeutically effective amount for at least one of the drugs in the mammal. The administration of the anti-diabetic drug can be before, during or after about the time the endogenous insulin level is at least maintained in the mammal.

In another embodiment, the method further includes discontinuing (either completely or partially) administration of at least one of the anti-diabetic drugs to the mammal. Cessation of the administration can be before, during or after about the time endogenous insulin production is at least maintained in the mammal. Preferably, the method is sufficient to prevent, treat, delay onset of, or at least alleviate symptoms associated with the diabetes in the mammal for a time after administration of the GLP-1, GLP-1 agonist or antidiabetic drug is discontinued.

As mentioned, practice of the invention can suitably prevent, treat, delay onset of or at least alleviate diabetic symptoms in subject mammals. In one embodiment, such benefits can be achieved in the mammal from about one day to about twenty five weeks. Of course, the length of the drug holiday period will depend on recognized parameters including the mammal of interest, the amount of GLP-1 and/or GLP-1 agonist administered, and the type of diabetes, if any, to be addressed. By way of example, administration of the GLP-1 or can be repeated as needed, eg., one or more times usually after (but sometimes before or during) the drug holiday period. Thus in a particular example, the administration of the GLP-1 or GLP-1 agonist is repeated one or more times after the time the endogenous insulin production at least maintained in the mammal.

Practice of the invention methods described herein is fully compatible with use of one or a combination of recognized anti-diabetic drugs including what is often referred to as a "cocktail" approach. Administration of such drugs can be before, during, or after a drug holiday although for most embodiments, the drugs will be given before or after a particular drug holiday. Use of the anti-diabetic drugs is not needed to practice the invention however it will be sometimes helpful to include such treatment in combination with use of GLP-1 and/or a GLP-1 agonist having GLP-1 like effect to manage disease in a particular subject.

For instance, and in one embodiment of the method, at least one of the anti-diabetic drugs is insulin, an insulin analog; or a pharmaceutically acceptable mixture thereof. Preferred human insulin is commercially available as HUMULIN^{™} and NOVULIN^{™}, for example. Additional insulins include bovine insulin,porcine insulin; or a mixture of insulins. Sometimes use of an insulin analog will be indicated. In such embodiments, the insulin analog of choice is Lys (B28), Pro (B29) human insulin.

The invention is further compatible with use of a wide spectrum of standard anti-diabetic drugs such as those disclosed by S. N. Davis and D. Granner, *supra*.

In one embodiment, the anti-diabetic drug is a sulfonylurea, biguanide, thiazolidinedione, diazoxide, somatostatin, or an alpha-glucosidase inhibitor such as acarbose. Prefered sulfonylureas according to the invention can be selected from the group consisting of tolbutamide, chlorpropamide, tolazamide, acetohexamide, glyburide, glipizide, and gliclazide. A particular biguanide of interest is metformin and phenformin. Suitable thiazolidinediones include ciglitazone and pioglitazone.

The present invention can be used to prevent or treat diabetes in a wide range of subject mammals. Use in a human patient that has, has had, is suspected of having, or who is pre-disposed to get diabetes will often be preferred. Typical diabetes will be diabetes mellitus or a related disorder. A preferred type of diabetes mellitus is selected from the group consisting of insulin-dependent diabetes millitus (IDDM or type I diabetes) and non-insulin-dependent diabetes mellitus (NIDDM, or type II diabetes). Examples of disorders related to diabetes mellitus have been described in S. N. Davis and D. Granner, *supra* and include, but are not limited to, impaired glucose tolerance (IGT); maturity-onset diabetes of youth (MODY); leprechaunism (insulin receptor mutation), tropical diabetes, diabetes secondary to a pancreatic disease or surgery; diabetes associated with a genetic syndome (eg., Prader-Willi syndrome); pancreatitis; and diabetes secondary to endocrinopathies; adipositas; and metabolic syndrome (syndroma X).

The present invention is related to specific observations discussed below and in the Examples. More specifically, two long-term studies were conducted in which *db*/*db* mice were dosed daily with a particular GLP-1 receptor agonist, COMPOUND 1. The first study was initiated to explore the dose-dependent anti-diabetic effect of COMPOUND 1 after intraperitoneal (i.p.) administration twice daily for 42 days. The second study was a conventional single-dose crossover study in which the animals were divided into two groups either dosed with COMPOUND 1 or vehicle. After 50 days, half of the vehicle-treated animals were switched to COMPOUND 1 treatment and half of the COMPOUND 1-treated animals were switched to vehicle treatment. In both studies, the progression of the type II diabetes was assessed by measuring the water- and food consumption, body weight, fasting blood glucose and glucose tolerance. To monitor the effect of COMPOUND 1 treatment on long-term control of blood glucose level, the amount of HbA_{1C} was measured at the end of the study. Furthermore, to address whether COMPOUND 1 mediates a direct protective effect on the β-cell function, the insulin mRNA expression was measured by RT-PCR as a marker for β-cells function.

As shown below, treatment of the animals with a new GLP-1 agonist improves insulin mRNA compared to a control (no active treatment). This improvement is seen for a long time period during the compound life time. When treatment with the agonist is stopped, the relatively high insulin mRNA level remains higher for a long period of time. This sustained boost in insulin message provided by the invention provides for the drug holiday.

Particular treatment of type II diabetes with GLP-1 agonists will improve insulin mRNA. This improvement has several benefits and will last for a long time ie., days, weeks or even months in some settings. This will allow human subjects to increase dosing intervals beyond the half-life of the compound. The duration of dose intervals compared to plasma half life can be evaluated eg., by measuring plasma insulin and plasma glucose response after a standard glucose intake before and after stopping dosing with the GLP-1 agonist. If desired, glycosylated haemoglobin can also be monitored.

The GLP-1 and GLP-1 agonists including derivatives and agonists thereof can be administered to a mammal in need of such treatment by one or several acceptable routes.

For instance, in type II diabetic patients Compound 1 can be administered subcutaneously. A wide range of formulation strategies are acceptable including use of recognized depot formulations or as water soluble formulations adapted for unitary dosing.

To better understand use of a particular GLP-1 or related molecule, subject mammals such as human patients can be divided into four groups. The study design is preferably a double blind randomized parallel study with a duration of about eight months. The three groups are dosed for one, three or six months after which dosing will be discontinued. The fourth group is treated with a placebo instead of the molecule to be tested. Fasting blood glucose, blood glucose response after a controlled oral glucose intake will be followed in parallel in all groups (particularly since it is a double blind design), in the days and weeks after stopping administration of the GLP-1 or related molecule. If desired, glycosylated haemoglobin can be followed as well. Parameters can be compared to the same parameters measured the last day before dosing with the molecule was stopped, with the other active treated groups, and with the parameters obtained in the placebo treated group. Preferred compounds such as Compound 1 have an effect on fasting blood glucose and blood glucose response after oral intake will last at least for days, preferably weeks after disappearance of the compound from the blood.

Plasma levels of the molecule to be tested can be measured using a conventional immunological approach such as RIA, Western blotting and/or ELISA.

Blood glucose and HbA1c can be measured by conventional standard bioanalytical methods.

A typically preferred administration strategy in accord with the invention is that GLP-1 and related molecules such as GLP-1 agonists (eg., Compound 1) shall be dosed only once every ten times or more of the biological half life, depending on recognized parameters such as the formulation type administered.

Progression of type II diabetes and related disorders can be monitored by one or a combination of suitable methods.

For example, type II patients are treated with at least one GLP-1 or a related molecule such a GLP-1 agonist and particularly Compound 1. Such treatment typically involves an individualized maximum tolerable dose of the compound either alone or in combination with at least one recognized anti-diabetic formulation. Examples of such formulations are disclosed herein and include oral antidiabetics such as glitazones, methformine, glucophages. It is typically useful to compare with a treatment matched patient group treated with placebo instead of the compound test.

Diagnostic tests for detecting and evaluating diabetes are known. Such tests include evaluation of peripheral neuropathy, typically by sense of vibration, retinopathy evaluated by ophtalmoscopic examination, myocardial ischaemia evaluated eg., by ECG, renal failure evaluated by proteinuria and measurements of glomerular filtration rate can be followed in an observation period of at least a few days, preferably a few weeks up to 1 to 3 years or more. The study is stopped when the compound of interest (eg., Compound 1) demonstrates a significantly slower development of secondary diabetic complications compared to non-treated patients.

Nearly any suitable statistical analysis treatment can be used. For instance, such an analysis can be made by analyzing absence of progression of the various symptoms ("survival analysis"). Suitably, at least one of peripheral neuropathy, retinopathy, myocardial ischaemia, renal loss of protein and renal insufficiency progress is significantly slower in patients receiving at least one of the GLP-1 or GLP-1 related molecule such as a GLP-1 agonist (eg., Compound 1). More specifically, treatment with the Compound 1, either alone or in combination with GLP-1 or a related molecule as described previously, will substantially reduce time of progression of type II diabetic complications.

The invention thus provides a highly useful method for providing blood glucose controlling therapy to a patient. In one embodiment, the method includes at least one and preferably all of the following steps: (a) administering to a patient in need of treatment at least one dose of at least one GLP-1 agonist in an amount sufficient to produce a therapuetically relevant plasma concentration of endogenously made insulin, (b) reducing or eliminating administration of the GLP-1 agonist from between about one day to about twenty five weeks, and (c) optionally repeating steps a) and b) sufficient to provide the insulin to the patient (drug holiday). Such a method can be combined, if desired, with nearly any of the standard anti-diabetic strategies as disclosed herein.

The Examples below show focus on results using COMPOUND 1. It is believed that the compound is a novel, rationally designed peptide GLP-1 receptor agonist that increases insulin release and improves glucose tolerance. COMPOUND 1 was characterized in two independent long-term studies in type II diabetic *db*/*db* mice as follows. *Study I:* Dose-response study. COMPOUND 1 was administered twice daily for 6 weeks at doses of 0, 1, 10 or 100 nmol/kg (n=10/group). *Study II:* Effect of COMPOUND 1 on β-cell preservation. Four groups of animals (n=15/group) were treated with vehicle (V) or COMPOUND 1 (100 nmol/kg; i.p.; once daily) in a cross-over design (50 + 40 days; groups: V+V, V+COMPOUND 1, COMPOUND 1+V, COMPOUND 1+COMPOUND 1).

Results of these studies are discussed below. Briefly, COMPOUND 1 effectively decreased fasting blood glucose (FBG). Blood glucose after an oral glucose load was significantly lower in COMPOUND 1 treated animals compared to controls. Glycosylated hemoglobin (HbA_{1c}) decreased dose-dependently (8.4±0.38% to 6.2±0.27%). In the V+V group, FBG, blood glucose after an oral glucose load, and HbA_{1c} levels were significantly higher than in mice treated with COMPOUND 1 throughout. Interestingly, these effects were preserved throughout the study in *db*/*db* mice treated with COMPOUND I only during the first 50 days of the study. The beneficial effects of early therapy with ZP10 were associated with an increased pancreatic insulin mRNA expression relative control animals.

Unless otherwise specified, the following abbreviations have been used: DMSO Dimethylsulphoxide; FBG Fasting blood glucose; HbA_{1C} Glycosylated hemoglobin; and OGTT Oral Glucose Tolerance Test

### EXAMPLE 1: GLP-1 and Compound 1 Bind GLP-1 Receptor

*Receptor binding studies*. These were carried out at MDS Panlabs, Panlabs Taiwan Ltd. In short, CHO-K1 cells harboring the human recombinant GLP-1 receptor were harvested. The membrane fraction was purified and used for binding assays. COMPOUND 1 and GLP-1 were solubilized in 0.4% DMSO. Membranes were incubated with different concentrations of test compounds covering 3 decades of concentrations in 20 mM Tris-HCl, pH 7.4, 5 mM MgCl₂, 20 mM NaCl, 1mM leupeptin, 1mM PMSF and 2% BSA for 90 min at 37°C in the presence of 0.03 nM ¹²⁵I-GLP-1 (7-36) amide. Radioactivity was measured in a γ-counter and IC₅₀-values were determined as the concentrations diminishing the specific binding (total binding minus non-specific binding in the presence of 100 nM GLP-1 (7-36) amide) by 50%.

*Binding to human GLP-1 receptors.* Concentrations resulting in half-maximal inhibition of binding to the human GLP-1 receptor expressed in CHO-K1 cells were 1.4 ± 0.24 nM and 5.5 ±1.3nM for COMPOUND 1 and GLP-1 (7-36) amide, respectively. Thus COMPOUND 1 was approximately 4 times more potent as an agonist than GLP-1 (7-36) amide.

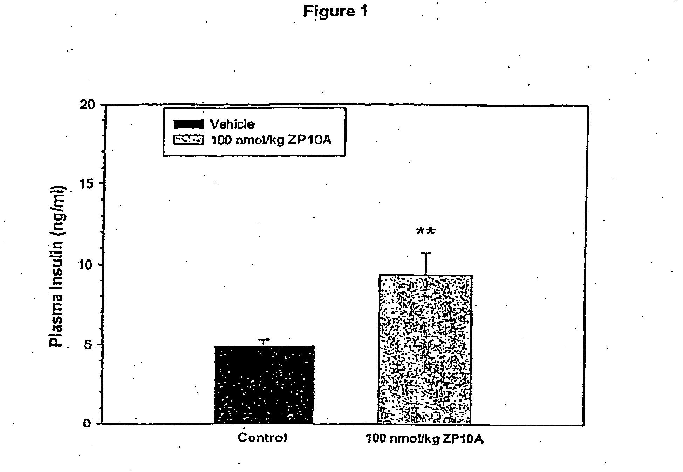

*Effect on plasma insulin levels*. In animals pre-treated with COMPOUND 1, 100 nmol/kg i.p., the oral glucose load produced an increase in plasma insulin levels that was about twice as high as the response observed in vehicle-treated animals (P = 0.002), **(****Figure 1****).**
**Figure 1** is explained in more detail as follows. It shows effect of COMPOUND 1 on release of insulin in *db*/*db* mice. Animals fasted overnight were given an oral glucose load of 1g/kg 15 min before receiving vehicle (n = 20) or 100 nmol/kg COMPOUND 1 (n = 19). Animals were bleed after 30 min and concentration of plasma insulin measured. (**: P = 0.002 vs. control animals).

### Example 2- Acute Effects Of Compound 1 On Glucose Tolerance.

The animals used were *db*/*db* mice 11-15 weeks old (M&B, Denmark). COMPOUND 1 was administered i.p. at doses of: 0.01, 0.1, 1, 10 and 100 nmol/kg (n=4-7/group) fifteen minutes before the animals were subjected to an oral glucose load (1 g/kg). Prior to the study, the area under the blood glucose concentration curve obtained over a 240-minute period (AUC₀₋₂₄₀; unit: mM·min) was used to stratify animals into five groups exhibiting similar glucose tolerances. Based on the dose-response relationship an ED₅₀ dose was estimated.

### Example 3: Effect Of Administering Compound 1 Over 42 Days

Animals included in this study were between 6 and 10 weeks old at the beginning of the study. Four days prior to the first dosing, the animals were weighed and subjected to an overnight fast (17 hrs). The fasted animals were then subjected to an oral glucose tolerance test (OGTT). The area under the blood glucose concentration curve obtained over a 240-minute period (AUC₀₋₂₄₀; unit: mM·min) was used to stratify animals into four groups exhibiting similar glucose tolerances. The animals were subjected to two daily i.p. doses of COMPOUND 1 at 8 am and 4 pm, respectively, for 42 days. Doses were 0 (vehicle), 1, 10 or 100 nmol/kg. The injection volume was 5 ml/kg in all groups.

*Parameters recorded in the 42 days study.* During the 42 days dosing period, body weight, food and water consumption were recorded daily. The animals over-night fasting blood glucose levels were measured on days -3, 1, 14, 41 and 43 and OGTT was performed on days -3, 1, 14 and 41 of the treatment period. On day 43 the animals were sacrificed and blood samples collected for measuring HbA_{1c}.

*Oral Glucose Tolerance Test (OGTT)*. To examine the effect of long-term treatment with COMPOUND I in the 42 days study, an OGTT was performed in connection with the morning dosing on days -3, 1, 14 and 41 of the 42 days dosing period. In the 90 days study an OGTT was performed in the morning on days 0, 50, 67, 78 and 90. Before the OGTT was performed, animals were subjected to an overnight fasting (17 hours). Blood samples were taken from the tip of the tail and blood glucose measured. The whole blood glucose (mM) concentration was analyzed by the immobilized glucose oxidase method using a drop of blood (< 5 µl; Elite Autoanalyser, Bayer, Denmark) following the manufacturer's manual. Blood samples containing glucose concentrations outside the measuring range of the Elite Autoanalyser was measured by an enzymatic/photometrical method at Nova Medical Medi-Lab A/S; Denmark. In the 42 days study the animals received daily dosing immediately after the initial blood sample (fasting blood glucose level), Fifteen minutes later (t = 0) an oral dose of glucose was administered (1 g/kg, 4 ml/kg) (Sigma, St. Louis, MO, U.S.A.) dissolved in a phosphate buffer (pH = 7.40). In the 90 days study, the oral glucose load was administered immediately after initial blood sampling. In both studies BG levels were measured at t=30 min, t=60 min, t=120 min and t=240 min. The area under the curve obtained over a 240-minute period (AUC₀₋₂₄₀; unit: mM·min) was included in the evaluation of the effect of the treatment.

*Dose response effect of COMPOUND 1 in the Oral Glucose Tolerance Test.* The dose-response relationship after acute i.p. administration of COMPOUND 1 demonstrated an ED₅₀ value of 0.021 nmol/kg.

*Dose-response effect of COMPOUND 1 in the 42 days study*. The body weight of the animals increased between 21.5% and 26.5% during the experiment (table 2). There was no statistically significant difference in weight gain between the COMPOUND 1 and the vehicle-treated group. However, a tendency toward a slightly higher weight gain could be detected in the animals receiving 100 nmol/kg COMPOUND 1 when compared to the vehicle-treated animals. The recorded water consumption revealed an extensive water intake in the vehicle-treated animals suggesting that the animals suffer from diabetes-induced polydipsia. Moreover, the daily water intake was reduced significantly and dose dependently in the mice treated with COMPOUND 1 (table 2; p<0.001 *vs*. vehicle).

**Table 2. Changes in body weight and water consumption at the end of the study period.**

| **42 days study** | △ Body weight | Water consumption |
|---|---|---|
| | (Mean ± SEM) | (Mean ± SEM) |
| Vehicle | 7.5 ± 1.2 | 36.2 ± 0.78 |
| 1 nmol/kg | 8.9 ± 1.1 | 21.7 ± 0.40* |
| 10 nmol/kg | 8.6 ± 1.8 | 16.2 ± 0.35* |
| 100 nmol/kg | 9.3 ± 1.1 | 14.0 ± 0.32* |

| **90 days study** | △ Body weight | Water consumption |
|---|---|---|
| | (Mean ± SEM) | (Mean ± SEM) |
| Vehicle-vehicle | 7.8 ± 1.4 | 31.7 ± 0.22 |
| Vehicle-ZP10 | 8.5 ± 1.2 | 14.7 ± 0.13* |
| ZP10-vehicle | 9.2 ± 1.6 | 21.6 ± 0.14* |
| ZP10-ZP10 | 9.0 ± 0.4 | 12.2 ± 0.09* |

| | | |
|---|---|---|
| *: p<0.05 vs. vehicle ***: p<0.05 vs. vehicle** | | |

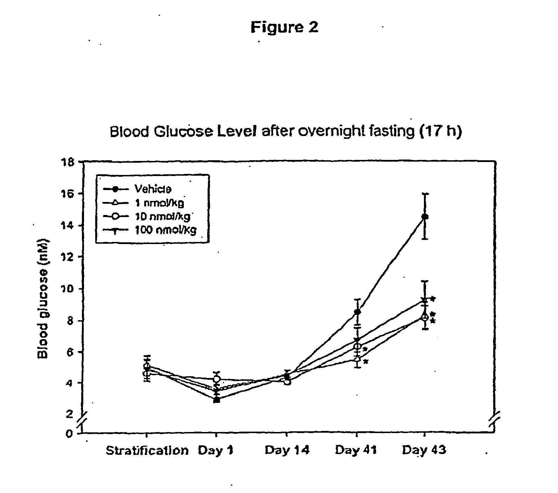

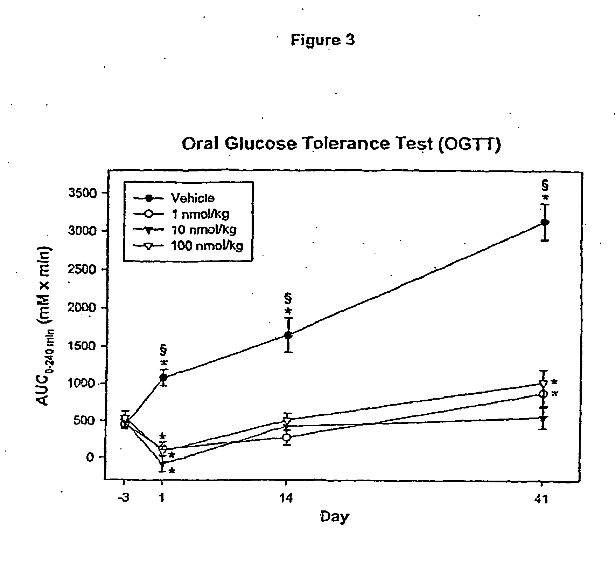

In the 42 days study, fasting blood glucose was measured on the day of stratification and during the study on day 1, 14, 41 and 43 (figure 2). On the day of stratification, on day 1 and day 14 no difference between the groups could be detected. However, on day 41 and 43 the fasting blood glucose level was significantly lower in the animals receiving COMPOUND 1, regardless of dose, suggesting that COMPOUND 1 elicited a marked antidiabetic effect. In order to further analyze the antihyperglycemic effect of COMPOUND 1 in both studies, the animals were subjected to an OGTT (figure 3). On the day of stratification all groups showed similar glucose tolerance. Interestingly, already after the very first dose of COMPOUND 1, glucose tolerance was improved in the ZP10 treated animals relative to vehicle-treated control animals. In vehicle-treated animals, the glucose tolerance was progressively impaired, and at the end of the study, this group showed a seven-fold decrease in their ability to respond to a glucose load when compared to the response in these animals at study start. In contrast, the COMPOUND 1 treated animals showed a clear improvement in their glucose response when compared to vehicle-treated animals. In fact, no significant change in the glucose tolerance could be detected at the end of the study when compared with the response to an OGTT on the day of stratification (figure 3).

Figure 2 is explained in more detail as follows. It shows fasting blood glucose concentrations on the day of stratification (day -3) and during treatment with COMPOUND 1 on day 1, 14 , 41 and 43. Mean ± SEM. *: p<0.05 vs. fasting BG concentration in vehicle-treated mice on the same day.

Figure 3 is discussed in more detail as follows. The figure shows oral Glucose Tolerance Test (OGTT) before treatment (day -3) and on day 1, 14 and 41 of long-term treatment with COMPOUND 1. Mean ± SEM. *: p<0.05 vs. AUC_{0-240 min} on day -3 within group. §: p<0.05 vs. AUC_{0-240 min} in all three ZP10-treated groups.

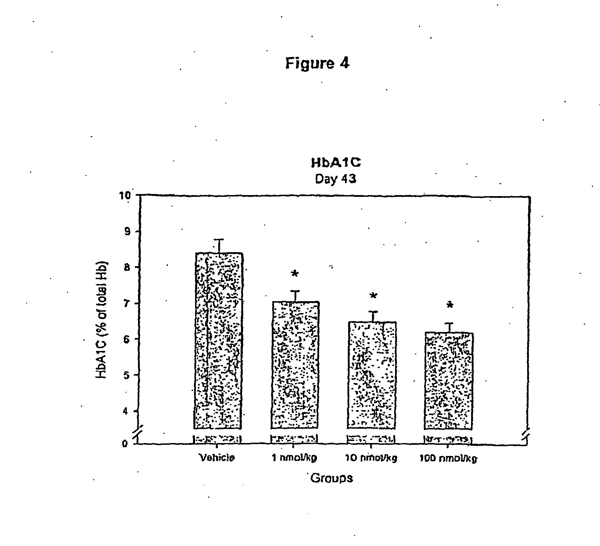

As an indicator of long-term blood glucose control, HbA_{1c} was measured at the end of the study (figure 4). The level of HbA_{1c} is expressed as a percentage of the total hemoglobin concentration. These data clearly show that long-term treatment with COMPOUND 1 significantly decreases the concentration of HbA_{1c} in a dose-dependent fashion. Figure 4 shows HbA_{1c} expressed as percent of total Hgb (hemoglobin) (42 days study). Data are mean ± SEM. *: p<0.01 vs. vehicle.

### Example 3: Effect Of Administering Compound 1 Over 90 Days

Three days prior to the first dosing, the animals were weighed and subjected to an overnight fast. The fasted animals were subjected to an OGTT (see below). The area under the blood glucose concentration curve obtained over a 240-minute period (AUC₀₋₂₄₀; unit: mM·min) was used to stratify animals into two groups exhibiting similar glucose tolerances. The animals were given one daily i.p. dose of COMPOUND 1, 100 nmol/kg or vehicle for a period of 50 days. The dosing was performed between 3 and 4 p.m. in order to ensure pharmacological efficacy during the period with maximal food intake, i.e. during night. After 50 days of dosing another OGTT was performed, and on this basis both the vehicle and the COMPOUND 1 treated group were re-stratified into four groups displaying similar glucose tolerances. Group 1, which initially received vehicle continued receiving vehicle. Group 2, which initially received vehicle was switched to COMPOUND 1 treatment (100 nmol/kg i.p.). Group 3, which initially received COMPOUND 1 was changed to vehicle treatment and group 4, which initially received COMPOUND 1, was continued on COMPOUND 1 treatment (100 nmol/kg i.p.). The treatment regimen is outlined in Table 1. This dosing regimen continued for 40 days.

**Table 1: Groups of treatment in the 90 days study**

| **Group** | **Days 1-50** | **Days 51-90** |
|---|---|---|
| 1 | Vehicle (n=21) | Vehicle (n=11) |
| 2 | | COMPOUND 1 (n=9*) |
| 3 | COMPOUND 1 (n=21) | Vehicle (n=11) |
| 4 | | COMPOUND 1 (n=10) |

| | | |
|---|---|---|
| ** One animal died on Day 71* | | |

*Parameters recorded in the 90 days study*. During the 90 days dosing period body weight and water consumption were recorded daily. The animals fasting blood glucose levels were measured on days 44, 58, 65, 72, 86 and 91 and an OGTT was performed on days 0, 50, 67, 78 and 90 of the treatment period. On day 91 the animals were sacrificed, blood samples collected for measuring glycosylated hemoglobin, and pancreas removed for Insulin mRNA measurements.

*Effect of COMPOUND 1 in the 90 days crossover study.* The results from the 42 days study strongly indicated that COMPOUND 1 delayed the progression of type II diabetes in *db*/*db* mice. However, it was unclear whether COMPOUND 1 preserved the β-cell function and thereby prevents the development of type I diabetes in these mice. Therefore, a 90 days cross-over study with a 50-day + 40-day study period was conducted. The animals were initially stratified and divided into two groups, one receiving vehicle and the other receiving 100 nmol/kg COMPOUND 1 i.p. once daily. After 50 days both groups were stratified again and divided into four groups. Group 1 continued receiving vehicle, group 2 initially received vehicle, but was changed to treatment with ZP10, group 3 initially received COMPOUND 1 and was changed to treatment with vehicle and finally group 4 continued receiving COMPOUND 1. The body weight of the animals was monitored during the study. Interestingly, during the 50 days no significant difference between the two initial groups could be detected (data not shown). However, after 90 days the body weight of group 3 and 4 was significantly higher than group 1 and 2 (table 2). This indicates that the general condition of the mice treated initially with COMPOUND 1 was better than the vehicle-treated group. The water consumption was also measured throughout the study and like in the 42 days study the water consumption was highest in the vehicle-treated groups. Interestingly, even after 40 days cessation of therapy with COMPOUND 1, the group treated with COMPOUND 1 during the first 50 days, still had lower water consumption than animals never treated with COMPOUND 1.

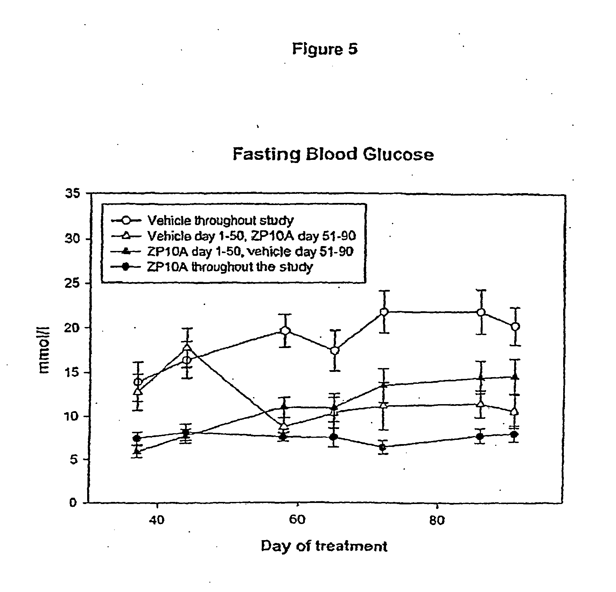

The fasting blood glucose was also measured during the study (figure 5). In the initial period from day 0-50, the fasting blood glucose level was significantly higher in the vehicle-treated animals than in the COMPOUND 1-treated animals. During the second treatment period, group 1 still displayed high fasting blood glucose. In contrast, group 2, 3 and 4, all had significantly lower fasting blood glucose levels throughout the 90 days study period. Group 2 exhibited an intermediate level of fasting blood glucose, but still significantly lower than group 1. These results indicate that these animals still have the ability to control their blood glucose levels after withdrawal of COMPOUND 1 for 40 days. Group 4 did not show any signs of hyperglycemia.

Figure 5 is explained in more detail as follows. It shows fasting blood glucose (FG) levels after eight hours of fasting. During Day 0-50, FG was significantly lower in animals treated with COMPOUND 1 compared with vehicle. Moreover, during the second treatment period (Days 51-90), FG was significantly higher in mice treated with vehicle throughout relative to the other three groups. However, mice that were changed from COMPOUND 1 to vehicle had a significant higher FG level than mice treated with COMPOUND 1 throughout.

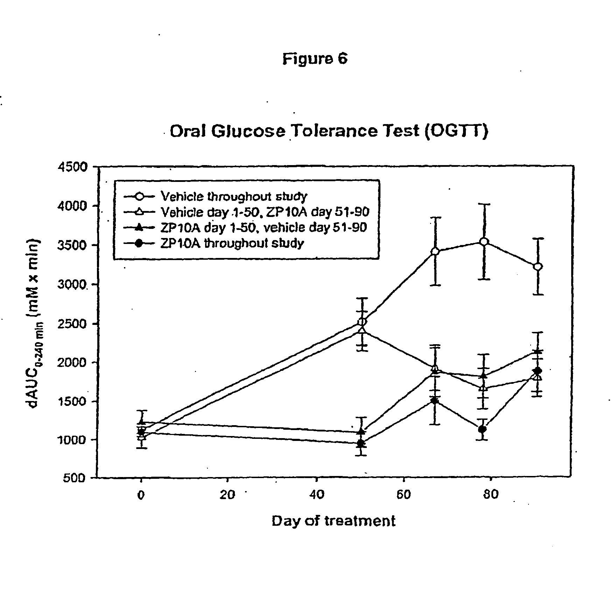

Oral glucose tolerance was measured five times during the study (figure 6). After the first 50 days the vehicle-treated group showed an impaired response to glucose whereas the glucose response of the COMPOUND 1-treated group did not deviate from the starting level. In fact, the glucose tolerances were similar in groups 2, 3 and 4 during the last 40 days. In group 3, glucose tolerance was significantly improved during the last period of the study in which the animals received treatment with COMPOUND 1 and at the end of the study, it was not different from glucose tolerances in groups 2 and 4.

Figure 6 is explained in more detail as follows. It shows oral Glucose Tolerance Test (OGTT) performed on Day 0, 50, 67, 78 and 90. Vehicle-treated *db*/*db* mice displayed progressively impaired glucose tolerance during the study. On Days 67-90, glucose tolerances were similar in the three groups of animals that were treated with COMPOUND 1 either during Day 1-50 (Group 2), Day 51-90 (Group 3), or throughout the entire study period (Group 4).

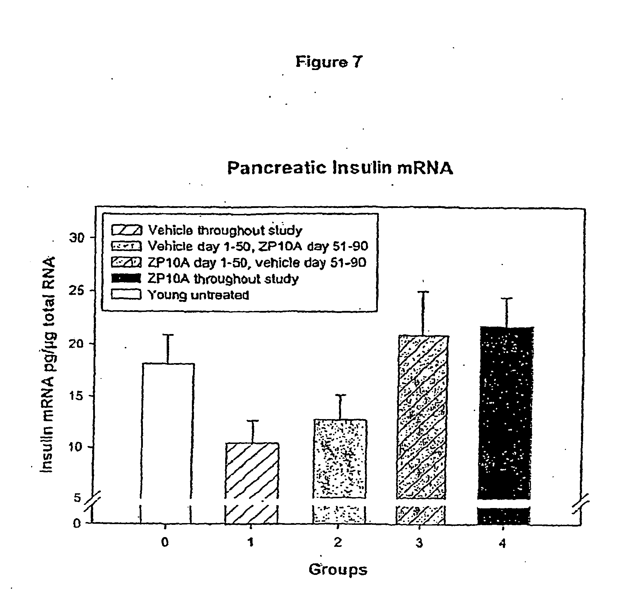

The prolonged effect on diabetic status after termination of COMPOUND 1 treatment (group 3) may reflect an improved β-cell function. In order to examine the β-cell function, we determined the expression of insulin mRNA at the end of the study (figure 7). Group 4 had increased expression of insulin mRNA compared to group 1. Intriguingly, the expression of insulin mRNA was similar in groups 3 and 4, indicating that early treatment with COMPOUND 1 prevents the deterioration of pancreatic insulin mRNA production. In order to evaluate the time-dependent changes in insulin mRNA expression following the respective treatments of groups 1-4, pancreatic insulin mRNA was also measured in a group of young untreated animals (6-10 weeks old; group 0 in figure 7). The results showed no significant difference between mRNA level in young animals and in animals treated with COMPOUND 1 for 50 or 90 days (groups 3 and 4).

Figure 7 is explained in more detail as follows. It shows a level of pancreatic insulin mRNA after the respective treatments. Mice treated with COMPOUND 1 throughout had an increased expression of insulin mRNA after 90 days of administration (Group 4) relative to vehicle-treated mice (Group 1). Interestingly, the expression of insulin mRNA was similar in mice treated with COMPOUND 1 during only the first 50 days and mice treated for 90 days, while animals in which treatment with COMPOUND 1 was not initiated until Day 50 showed an expression of insulin that was similar to the expression found in vehicle-treated mice. The level of insulin mRNA was similar in young untreated mice (group 0) and in the two groups that had received COMPOUND 1 treatment during the first part of the study (groups 3 and 4).

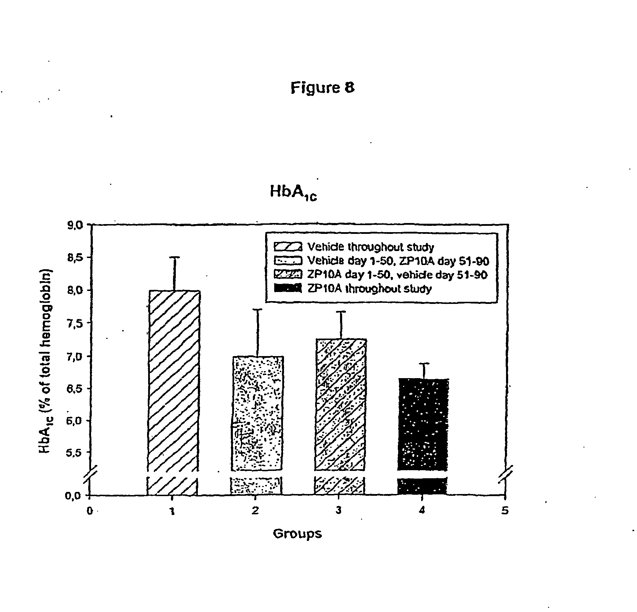

Finally, the HbA_{1c} levels were measured at the end of the study (figure 8). The three groups that received COMPOUND 1 treatment had lower levels of HbA_{1c} than animals receiving vehicle treatment throughout, but the differences did not reach statistical significance.

Figure 8 is explained in more detail as follows. It shows: HbA_{1c} levels (% of total hemoglobin concentration) measured on the day of termination. One-way ANOVA showed no overall significant difference among groups (p = 0.22). However, Fisher's LSD test for posthoc comparisons showed that HbA_{1c} was significantly lower in mice treated with COMPOUND 1 throughout (Group 4: 6.65±0.22%) relative to vehicle-treated mice (Group 1: 7.99±0.51%).

### Example 4: Effect of COMPOUND 1 On Insulin Release In db/db Mice.

To examine the effect of COMPOUND 1 on physiological insulin release during hyperglycemia, insulin levels were determined after an oral glucose load (1 g/kg). Thirty nine overnight fasted animals entered the experiment one week after being stratified into two group after an OGTT as described above. Fifteen minutes before the animals were given an oral glucose load, each animal received vehicle or 100 nmol/kg COMPOUND 1 i.p. Thirty minutes after the glucose administration, the animals were bled by left ventricular puncture during carbon dioxide anaesthesia. The blood was collected using a syringe mounted with a needle pre-flushed with heparine (5000 i.u./ml). The blood samples were quickly transferred into a pre-chilled test tube that contained 5 µl 0.5 M EDTA and 5 µl Trasylol^{®} (aprotinin, 20 x 10⁻⁶ IU/ml) and centrifuged at 3000 rpm for 10 minutes at 2-4 °C. Plasma was kept cold during harvest, frozen on dry ice and stored at -80°C for later analysis of hormones. The whole blood glucose (mM) concentration was analysed as described above. Plasma concentrations of insulin were measured in samples of 10 µl plasma using a commercial enzyme immunoassay from Peninsula Laboratories Europe, LTD (ELIS7537).

Unless otherwise noted, the following materials and methods were used, as needed, in the Examples discussed above.

*Animals*. Male *db*/*db* mice C57BLKS/J-Leprdb/Leprdb weighing 37.3 ± 1.1 g (M&B, Ll. Skensved, Denmark) at time of inclusion were used. The mice were housed (3 mice/cage) under controlled conditions (20°C, 55-85% humidity) following a 12:12-h light/dark cycle with light on at 6 am. The animals were fed ad libitum with a standard Altromin No. 1324 diet (Chr. Petersen, Ringsted, Denmark) and had free access to domestic quality tap water. At the time of inclusion, all mice had overnight fasting (17 hrs) blood glucose (BG) levels below 10 mmol/l. No animals included in the study displayed blood glucose levels above 33 mM when subjected to a standard oral glucose load (see below).

*Water Consumption*. At the time of the morning dosing the animals were weighed, and water consumption per group was measured gravimetrically by weighing the water bottle and calculating the amount of water consumed.

*Isolation of total RNA from mice pancreas*. The frozen pancreatic glands were weighed and minced in a mortar under liquid nitrogen. The extraction of total RNA was conducted as described by the manufacturer of the kit(Qiagen Rneasy kit, VWR International).

*First strand synthesis*. 0.5-1.0 µg total RNA was used for first strand synthesis. In brief, the RNA was incubated for 10 min at 70°C and quenched on ice. The RNA was equilibrated to 42°C and mixed with 10mM dNTP, lul Superscript II (Life Technologies) in a final volume of 20 µl and incubated for an additional hour at 42°C. The reaction was terminated by incubation for 5 min at 94°C.

*Insulin standard for quantitative PCR*. One µl first strand synthesis was used for PCR with the following insulin primers: 5'-AACCCACCCAGGCTTTTGTCA; 5'-CTTCCTCCCACGTCCAGTTGTTC-3 The amplicon were inserted into the PCR 4-TOPO vector (invitrogen) and transformed into E.coli. The plasmids were purified and 2 µg of each were linearized with either Spe I or Not I. The linearized plasmids were *in-vitro* transcribed using T7 or T3 RNA polymerase. After *in-vitro* transcription, the template was removed by DNAse treatment. Subsequently, the mixture was phenol/chloroform-extracted and precipitated. After precipitation the RNA was dissolved to 1 mg/ml in water.

*Quantitative PCR*. One µg of both standard and sample were subjected to first strand synthesis as described above. A dilution series of the insulin mRNA standard, together with the samples were subjected to quantitative PCR using the following probe (Mouse insulin Taqman probe, 110-138) and the above described primers:
5'-FAM-AGGCTCTCTACCTGGTGTGTGGGGAGCGT- Tamra-3'
All PCR reactions were duplicates. The Cₜ (threshold cycle) were measured and the initial concentration of insulin mRNA was calculated according to the standard curve.

*Drugs:* COMPOUND 1 (H-HGEGTFTSDLSKQMEEEAVRLFIEWLKNGGPSSGAPPSK KKKKK-NH2, Batch: ZP15.65-3A) was produced at Zealand Pharma A/S using the Merifield technique.

*Statistics:* One-way classified data were analysed using one-way ANOVA and Fisher's LSD test for post-hoc analysis. Two-way classified data were analysed using a two-way ANOVA. Unpaired data were analysed using the Student's t-test for unpaired data.

The foregoing Examples show that COMPOUND 1 was a highly efficacious GLP-1 agonist with potent anti-diabetic efficacy. It binds to the human GLP-1 receptor with an affinity 4 times higher than GLP-1 itself, it potentiates the secretion of insulin in response to an oral glucose load, and normalizes glucose intolerance in diabetic *db*/*db* mice at doses in the low nmol/kg range. Furthermore, after prolonged treatment, COMPOUND 1 increases the insulin mRNA level, and normalizes HbA_{1c} levels. The Examples show that prolonged treatment with COMPOUND 1 reduces progression of type II diabetes in *db*/*db* mice.

In this Example, all three doses of COMPOUND 1 produced a similar improvement of glucose tolerance, fasting blood glucose and HbA_{1C} suggesting that COMPOUND 1 produces a maximal antidiabetic response during long-term administration of doses from 1-100 nmol/kg i.p. The improved glucose tolerance was closely related to a decrease in daily water intake in the COMPOUND 1 treated mice. Moreover, the daily water intake was significantly lower in the animals receiving 100 nmol/kg than in both vehicle-treated mice and in animals treated with only 1 nmol/kg of COMPOUND 1. These results are consistent with the clinical finding that thirst and polydipsia are closely related to blood glucose levels in diabetic subjects.

Animals treated with vehicle exhibited a high level of HbA_{1c} in contrast to the clear dose-dependent decrease shown in the groups treated with COMPOUND 1. Without wishing to be bound to theory, the data in this Example is consistent with COMPOUND 1 exerting a protective effect on the pancreas (direct or indirect) that can postpone the development of severe diabetes associated with ketoacidosis and early death in *db*/*db* mice.

This question was addressed in the Examples. Specifically, a cross-over study was designed in which animals were changed from treatment with COMPOUND 1 to vehicle. In the 42 days study, it was found that 100 nmol/kg was the most effective dose. Thus, 100 nmol/kg was used as a single daily injection in the cross-over study. The major finding of this study was that three months treatment with COMPOUND 1 prevented the progressive development of diabetes in *db*/*db* mice. Ninety days of COMPOUND 1 treatment increased glucose tolerance, decreased fasting glucose level, decreased HbA_{1C}, decreased water intake, and increased the expression of insulin mRNA in pancreatic β-cells relative to vehicle-treated control mice.

Without wishing to be bound to theory, the increased expression of pancreatic insulin mRNA suggests that the improved glucose tolerance in COMPOUND 1 treated *db*/*db* mice was related to an improved ability to release of insulin in response to oral glucose load. This observation is supported by the Examples above, particulary the finding that plasma insulin levels increased twice as much in response to an oral glucose load in COMPOUND 1 treated mice than in untreated control animals.

Interestingly, in mice treated with COMPOUND 1 during the first 50 days of the study period, the COMPOUND 1 treatment produced a sustained improvement in glucose tolerance, decreased fasting glucose levels, lower water intake, and an elevated expression of insulin mRNA compared to vehicle-treated animals. These results demonstrate that i.p. administration of COMPOUND 1 once daily effectively prevents the progression of diabetes in *db*/*db* mice. Without wishing to be bound by theory, the sustained effect on glucose metabolism and pancreatic expression of insulin mRNA is consistent with Compound 1 preserving β-cell function in diabetic *db*/*db* mice. The persistent effect of COMPOUND 1 on insulin mRNA in the group that was shifted to vehicle (placebo) indicates a β-cell sparing mechanism and/or neogenesis of β-cells from progenitor cells. There was no significant difference between insulin mRNA level in young animals and in animals treated with COMPOUND 1 for 50 or 90 days (groups 3 and 4) indicating that the treatment protects the β-cells and significantly delays the progression of the type II diabetes. In mice treated with vehicle during the first treatment period and with COMPOUND 1 from day 51 and onward, glucose tolerance, fasting blood glucose and water intake were improved during therapy with COMPOUND 1. Late onset of therapy with COMPOUND 1 did not substantially improve the expression of insulin mRNA in pancreatic β-cells if treatment was initiated half-way through the study.

The Examples also show that the protective action of COMPOUND 1 is most effective when therapy is initiated early during the diabetic development. When therapy with COMPOUND 1 was initiated in animals with late untreated diabetes, COMPOUND 1 improved glucose tolerance in absence of changes in the expression of insulin mRNA. The presence of detectable insulin mRNA in vehicle-treated mice suggests that even these severely ill animals were able to produce insulin.

The Examples further show that Compound 1 is an effective anti-diabetic compound that prevents the progression of diabetes in *db*/*db* mice. Without wishing to be bound to theory, the sustained effect on glucose metabolism, and pancreatic expression of insulin after discontinuation of COMPOUND 1 treatment indicates that COMPOUND 1 preserves β-cell function in diabetic *db*/*db* mice.

It was found that COMPOUND 1 is an effective anti-diabetic compound that prevents the progression of diabetes in *db*/*db* mice. The sustained effect on glucose metabolism, and pancreatic expression of insulin mRNA after discontinuation of COMPOUND 1 treatment indicates that COMPOUND 1 preserves β-cell function in diabetic *db*/*db* mice.

The Examples can be further summarized as follows.

Compound 1 is a new and rationally designed GLP-1 agonist. COMPOUND 1 is a peptide with a high affinity for the GLP-1 receptor that increases insulin release and improves glucose tolerance. The pharmacological efficacy of COMPOUND 1 was characterized in two independent long-term studies in type 11 diabetic db/db mice lasting 6 and 12 weeks, respectively. In the 6-week study, COMPOUND 1 was administered twice daily for 6 weeks at doses of 0, 1,10 or 100 nmol/kg (n=10/group). This study demonstrated that COMPOUND 1 effectively decreased fasting blood glucose (FBG) from 13.7±1.3 mM in control animals to 8.6±1.4 mM in COMPOUND 1 treated groups. While glucose tolerance remained unchanged during the 6-week study period for all COMPOUND 1 treated animals, the area under the curve after an oral glucose load increased 5-6 fold at the end of the study in vehicle-treated (placebo) mice. After 6 weeks, glycosylated hemoglobin (HbA, j decreased dose-dependently from 8.4±0.38% in control animals to 6.21±0.27% in mice treated with 100 nmol/kg COMPOUND 1. In order to assess if the long-term effects of COMPOUND 1 (100 nmol/kg; i.p.; once daily) were mediated by a Beta-cell preserving mechanism, 4 groups of animals (n=15/group) were treated with vehicle (V) or COMPOUND 1 in a cross-over design (50 + 40 days; groups: V+V, V+COMPOUND 1, COMPOUND 1+V. COMPOUND 1+COMPOUND 1). In the V+V group, FBG, blood glucose during an oral glucose tolerance test (OGTT), and HbA_{1c}, levels were significantly higher than in mice treated with COMPOUND 1 throughout. Interestingly, in db/db mice treated only during the first 50 days of the study, FBG, OGTT response, and HbAj, was still lower than in V+V after 90 days. The beneficial effects of early therapy with ZP10 were associated with an increased pancreatic insulin mRNA expression relative to untreated diabetic mice (V+V: 10.4±2.2, V+COMPOUND 1: 12.7±2.4; COMPOUND 1+V: 20.9±4.1; COMPOUND 1+COMPOUND 1: 21.8±2.7 pg/mg total RNA). These studies demonstrate that COMPOUND 1 is an effective antidiabetic compound that prevents the progression of diabetes in db/db mice. Without wishing to be bound to any theory, it is believed that sustained effect on glucose metabolism, and pancreatic expression of insulin after discontinuation of COMPOUND 1 treatmxent indicates that COMPOUND 1 preserves β-cell function in diabetic db/db mice.
1. Holst,JJ (1999) Glucagon-like peplide-1, a gastrointestinal hormone with a pharmaceutical potential. Curr Med Chem. 6:1005-1017
2. Nauck,MA, Holst,JJ, Willms,B, Schmiegel,W (1997) Glucagon-like peptide 1 (GLP-1) as a new therapeutic approach for type 2-diabetes. Exp Clin Endocrinol Diabetes 105:187-195
3. Lopez-Delgado,MI, Morales,M, Villanueva-Penacarrillo,ML, Malaisse,WJ, Valverde,I (1998) Effects of glucagon-like peptide 1 on the kinetics of glycogen synthase a in hepatocytcs from normal and diabetic rats. Endocrinology 139:2811-2817
4. Byme,MM, Gliem,K, Wank,U, Arnold,R, Katschinski,M, Polonsky,KS, Goke,B (1998) Glucagon-like peptide I improves the ability of the beta-cell to sense and respond to glucose in subjects with impaired glucose tolerance. Diabetes 47:1259-1265
5. Raufman,JP, Singh,L, Singh,G, Eng,J (1992) Truncated glucagon-like peptide-1 interacts with exendin receptors on dispersed acini from guinea pig pancreas. Identification of a mammalian analogue of the reptilian peptide exendin-4. J Biol Chem 267:21432-7
6. Young,AA, Gedulin,BR, Bhavsar,S, Bodkin,N, Jodka,C, Hansen,B, Denaro,M (1999) Glucose-lowering and insulin-sensitizing actions of exendin-4: studies in obese diabetic (ob/ob, db/db) mice, diabetic fatty Zucker rats, and diabetic rhesus monkeys (Macaca mulatta). Diabetes 48:1026-1034
7. Edvell,A, Lindström,P (1999) Initiation of increased pancreatic islet growth in young normoglycemic mice (Umeå +/?). Endocrinology 140:778-783
8. Xu,G, Stoffers,DA, Habener,JF, Bonner-Weir,S (1999) Exendin-4 stimulates both beta-cell replication and neogenesis, resulting in increased beta-cell mass and improved glucose tolerance in diabetic rats. Diabetes 48:2270-2276
9. Greig,NH, Holloway,HW, De Ore,KA, Jani,D, Wang,Y, Garant,MJ, Egan,JM (1999) Once daily injection of exendin-4 to diabetic mice achieves long-term beneficial effects on blood glucose concentrations. Diabetologia 42:45-50
10. Parkes,DG, Pittner,R, Jodka,C, Smith,P, Young,A (2001) Insulinotropic actions of exendin-4 and glucagon-like peptide-1 in vivo and in vitro. Metabolism 50:583-589
11. Chen,H, Charlat,O, Tartaglia,LA, Woolf,EA, Wang,X, Ellis,SJ, Lakey,ND, Culpepper,J, Moore,KJ, Breitbart,RE, Duyk,GM, Tepper,RI, Morgenstem,JP (1996) Evidence that the diabetes gene encodes the leptin receptor: identification of a mutation in the leptin receptor gene in db/db mice. Cell 84:491-495
12. Coleman,DL (1973) Effects of parabiosis of obese with diabetes and normal mice. Diabetologia 9:294-298
13. Leiter,EH, Coleman,DL, Ingram,DK, Reynolds,MA (1983) Influence of dietary carbohydrate on the induction of diabetes in C57BL/KsJ-db/db diabetes mice. J.Nutr. 113:184-195
14. Hui,H, Wright,C, Perfetti,R (2001) Glucagon-like peptide 1 induces differentiation of islet duodenal homeobox-1-positive pancreatic ductal cells into insulin-secreting cells. Diabetes 50:785-796
15. Tourrel,C, Bailbe,D, Meile,MJ, Kergoat,M, Portha,B (2001) Glucagon-like peptide-1 and exendin-4 stimulate beta-cell neogenesis in streptozotocin-treated newborn rats resulting in persistently improved glucose homeostasis at adult age. Diabetes 50:1562-1570
16. USSN 60/393,917 by E. Steiness entitled "Method For Treating Diabetes and Related Disorders" as filed on July 4, 2002.

## Claims

1. Use of at least one GLP-1 or a GLP-1 agonist having GLP-1 effect for the
manufacture of a medicament for use in a method of preventing or treating diabetes in a mammal, wherein the method comprises reducing administration of the GLP-1 or GLP-1 agonist below about the therapeutically effective amount for a time conducive
to producing a drug holiday, wherein the drug holiday is further defined as a time interval between a first endpoint following the reduction in administering the GLP-1 or GLP-1 agonist and a second endpoint.

2. A GLP-1 or a GLP-1 agonist having GLP-1 effect, for use in a method of treatment or prevention of diabetes in a mammal, the method comprising reducing administration of the GLP-1 or GLP-1 agonist below about the therapeutically effective amount for a time conducive to producing a drug holiday, wherein the drug holiday is further defined as a time interval between a first endpoint following the reduction.in administering the GLP-1 or GLP-1 agonist and a second endpoint.

3. The use according to claim 1 or the GLP-1 or GLP-1 agonist according to claim 2, wherein:
(a) administration of the GLP-1 or GLP-1 agonist is reduced during the drug holiday by at least about 50% below the therapeutic amount; or
(b) administration of the GLP-1 or GLP-1 agonist is reduced during the drug holiday by at least about 90% below the therapeutic amount.

4. The use according to claim 1 or claim 3 or the GLP-1 or GLP-1 agonist according to claim 2 or claim 3, wherein administration of the GLP-1 or GLP-1 agonist is stopped during the drug holiday.

5. The use according to claim 1 or the GLP-1 or GLP-1 agonist according to claim 2, wherein the second endpoint is identified by a standard FBG or glycosylated hemoglobin test.

6. The use according to any one of claims 1 and 3 to 5 or the GLP-1 or GLP-1 agonist according to any one of claims 2 to 5, wherein:
(a) the drug holiday is for about one day to about twenty-five weeks; or
(b) the drug holiday is for between from about three to four weeks.

7. The use according to any one of claims 1 and 3 to 6 or the GLP-1 or GLP-1 agonist according to any one of claims 2 to 6, wherein:
(a) the GLP-1 or GLP-1 agonist is administered as a depot formulation; or
(b) the GLP-1 or GLP-1 agonist is administered to the mammal bolus at least about once daily; or
(c) the GLP-1 or GLP-1 agonist is administered to the mammal bolus at least once a week; or
(d) the administration of the GLP-1 or GLP-1 agonist is about twice daily (i.v. or subQ) for between from about one to about twenty weeks.

8. The use according to any one of claims 1 and 3 to 7 or the GLP-1 or GLP-1 agonist according to any one of claims 2 to 7, the method further comprising administering to the mammal a second therapeutically effective amount of GLP-1 or GLP-1 agonist following the drug holiday.

9. The use or the GLP-1 or GLP-1 agonist according to claim 8, the method further comprising reducing administration of the second therapeutically effective amount of GLP-1 or GLP-1 agonist for a time conducive to producing a second drug holiday.

10. The use according to any one of claims 1 and 3 to 9 or the GLP-1 or GLP-1 agonist according to any one of claims 2 to 9, wherein in the method:
(a) the administration and reducing steps are repeated at least once; or
(b) the administration and reducing steps are repeated at least about 2 to about 25 times; or
(c) the administration and reducing steps are repeated as needed to prevent or treat the diabetes or related disorder.

11. The use or the GLP-1 or GLP-1 agonist according to claim 10, wherein the method is practised over the lifetime of the mammal.

12. The use according to any one of claims 1 and 3 to 11 or the GLP-1 or GLP-1 agonist according to any one of claims 2 to 11, wherein the GLP-1 or GLP-1 agonist is administered to the mammal at a dose of at least about 0.01 nmol/kg (body weight).

13. The use according to any one of claims 1 and 3 to 12 or the GLP-1 or GLP-1. agonist according to any one of claims 2 to 12, wherein the GLP-1 or GLP-1 agonist is exendin-4, exendin-3; or an analog or derivative thereof.

14. The use according to any one of claims 1 and 3 to 13 or the GLP-1 or GLP-1 agonist according to any one of claims 2 to 13, wherein the medicament is administered with at least one anti-diabetic drug.

15. The use or the GLP-1 or GLP-1 agonist according to claim 14, wherein:
(a) the administration is below about a therapeutically effective amount for at least one of the GLP-1 or GLP-1 agonist, or the anti-diabetic drug in the mammal; or
(b) the administration is at least about at a therapeutically effective amount for at least one of the GLP-1 or GLP-1 agonist, or the anti-diabetic drug in the mammal.

16. The use according to any one of claims 1 any 3 to 15 or the GLP-1 or GLP-1 agonist according to any one of claims 2 to 15, wherein administration of the antidiabetic drug is before or after the drug holiday.

17. The use according to any one of claims 1 and 3 to 16 or the GLP-1 or GLP-1 agonist according to any one of claims 2 to 16, wherein at least one of the antidiabetic drugs is insulin, an insulin analogue; or a pharmaceutically acceptable mixture thereof.

18. The use or the GLP-1 or GLP-1 agonist according to claim 17, wherein:
(a) the insulin is human insulin, bovine insulin, porcine insulin; or a mixture thereof; or
(b) the insulin analogue is Lys (B28), Pro (B29) human insulin; or
(c) the anti-diabetic drug is a sulfonylurea, biguanide, thiazolidinedione, diazoxide, somatostatin, or an alpha-glucosidase inhibitor.

19. The use or the GLP-1 or GLP-1 agonist according to claim 18, wherein:
(a) the sulfonylurea is selected from the group consisting of tolbutamide, chlorpropamide, tolazamide, acetohexamide, glyburide, glipizide, and gliclazide; or
(b) the biguanide is metformin or phenformin; or
(c) the thiazolidinedione is ciglitazone or pioglitazone; or
(d) the alpha-glucosidase inhibitor is acarbose.

20. The use according to any one of claims 1 and 3 to 19 or the GLP-1 or GLP-1 agonist according to any one of claims 2 to 19, wherein the mammal is a human subject who has or is suspected of having diabetes mellitus or a related disorder.

21. The use or the GLP-1 or GLP-1 agonist according to claim 20, wherein:
(a) the diabetes mellitus is selected from the group consisting of insulin-dependent diabetes millitus (IDDM or type I diabetes) and non-insulin-dependent diabetes mellitus (NIDDM, or type II diabetes); or
(b) the human subject suspected of having the diabetes mellitus is genetically pre-disposed to develop the disease; or
(c) the disorder related to diabetes mellitus is selected from the group consisting of impaired glucose tolerance (IGT), maturity-onset diabetes of youth (MODY); leprechaunism (insulin receptor mutation), tropical diabetes, diabetes secondary to a pancreatic disease or surgery; diabetes associated with a genetic syndome (eg., Prader-Willi syndrome); pancreatitis; and diabetes secondary to endocrinopathies; adipositas; and metabolic syndrome (syndrome X).

## Patentansprüche

1. Verwendung von zumindest einem GLP-1 oder GLP-1-Agonisten mit GLP-1-Wirkung zur Herstellung eines Medikaments zur Verwendung in einem Verfahren zur Prävention oder Behandlung von Diabetes bei einem Säugetier, worin das Verfahren die Reduktion der Verabreichung des GLP-1 oder des GLP-1-Agonisten auf eine Menge unter etwa der therapeutisch wirksamen Menge für eine Zeitspanne umfasst, die für die Herbeiführung einer arzneimittelfreien Zeit förderlich ist, worin die arzneimittelfreie Zeit weiters als eine Zeitspanne zwischen einem ersten Endpunkt nach der Reduktion der Verabreichung des GLP-1 oder des GLP-1-Agonisten und einem zweiten Endpunkt definiert ist.

2. GLP-1 oder GLP-1-Agonist mit GLP-1-Wirkung zur Verwendung in einem Behandlungs- oder Präventionsverfahren von/für Diabetes bei einem Säugetier, wobei das Verfahren die Reduktion der Verabreichung des GLP-1 oder des GLP-1-Agonisten auf eine Menge unter etwa der therapeutisch wirksamen Menge für eine Zeitspanne umfasst, die für die Herbeiführung einer arzneimittelfreien Zeit förderlich ist, worin die arzneimittelfreie Zeit weiters als eine Zeitspanne zwischen einem ersten Endpunkt nach der Reduktion der Verabreichung des GLP-1 oder des GLP-1-Agonisten und einem zweiten Endpunkt definiert ist.

3. Verwendung nach Anspruch 1 oder GLP-1 oder GLP-1-Agonist nach Anspruch 2, worin:
(a) die Verabreichung des GLP-1 oder GLP-1-Agonisten während der arzneimittelfreien Zeit um zumindest etwa 50 % unter die therapeutische Menge reduziert wird; oder
(b) die Verabreichung des GLP-1 oder GLP-1-Agonisten während der arzneimittelfreien Zeit um zumindest etwa 90 % unter die therapeutische Menge reduziert wird.

4. Verwendung nach Anspruch 1 oder Anspruch 3 oder GLP-1 oder GLP-1-Agonist nach Anspruch 2 oder Anspruch 3, worin die Verabreichung des GLP-1 oder GLP-1-Agonisten während der arzneimittelfreien Zeit ausgesetzt wird.

5. Verwendung nach Anspruch 1 oder GLP-1 oder GLP-1-Agonist nach Anspruch 2, worin der zweite Endpunkt durch einen Standard-FBG- oder einen Glykosyliertes-Hämoglobin-Test identifiziert wird.

6. Verwendung nach einem der Ansprüche 1 und 3 bis 5 oder GLP-1 oder GLP-1-Agonist nach einem der Ansprüche 2 bis 5, worin:
(a) die arzneimittelfreie Zeit etwa einen Tag bis etwa fünfundzwanzig Wochen beträgt; oder
(b) die arzneimittelfreie Zeit etwa drei bis vier Wochen beträgt.

7. Verwendung nach einem der Ansprüche 1 und 3 bis 6 oder GLP-1 oder GLP-1-Agonist nach einem der Ansprüche 2 bis 6, worin:
(a) das GLP-1 oder der GLP-1-Agonist als eine Depot-Formulierung verabreicht wird; oder
(b) das GLP-1 oder der GLP-1-Agonist dem Säugetier als Bolus zumindest etwa einmal täglich verabreicht wird; oder
(c) das GLP-1 oder der GLP-1-Agonist dem Säugetier als Bolus zumindest etwa einmal pro Woche verabreicht wird; oder
(d) die Verabreichung des GLP-1 oder GLP-1-Agonisten etwa zwei Mal täglich (i.v. oder subkutan) für eine Zeitspanne von zwischen etwa einer und etwa zwanzig Wochen erfolgt.

8. Verwendung nach einem der Ansprüche 1 und 3 bis 7 oder GLP-1 oder GLP-1-Agonist nach einem der Ansprüche 2 bis 7, wobei das Verfahren weiters die Verabreichung einer zweiten therapeutisch wirksamen Menge von GLP-1 oder GLP-1-Agonist nach der arzneimittelfreien Zeit umfasst.

9. Verwendung oder GLP-1 oder GLP-1-Agonist nach Anspruch 8, wobei das Verfahren weiters die Reduktion der Verabreichung der zweiten therapeutisch wirksamen Menge des GLP-1 oder GLP-1-Agonisten für eine Zeitspanne umfasst, die für die Herbeiführung einer zweiten arzneimittelfreien Zeit förderlich ist.

10. Verwendung nach einem der Ansprüche 1 und 3 bis 9 oder GLP-1 oder GLP-1-Agonist nach einem der Ansprüche 2 bis 9, worin in dem Verfahren:
(a) die Verabreichungs- und Reduktionsschritte zumindest einmal wiederholt werden; oder
(b) die Verabreichungs- und Reduktionsschritte zumindest etwa 2- bis etwa 25-mal wiederholt werden; oder
(c) die Verabreichungs- und Reduktionsschritte so oft wie nötig wiederholt werden, um Diabetes oder eine verwandte Erkrankung zu verhindern oder zu behandeln.

11. Verwendung des GLP-1 oder GLP-1-Agonisten nach Anspruch 10, worin das Verfahren während der Lebenszeit des Säugetiers praktiziert wird.

12. Verwendung nach einem der Ansprüche 1 und 3 bis 11 oder GLP-1 oder GLP-1-Agonist nach einem der Ansprüche 2 bis 11, worin das GLP-1 oder der GLP-1-Agonist dem Säugetier in einer Dosis von zumindest etwa 0,01 nmol/kg (Körpergewicht) verabreicht wird.

13. Verwendung nach einem der Ansprüche 1 und 3 bis 12 oder GLP-1 oder GLP-1-Agonist nach einem der Ansprüche 2 bis 12, worin das GLP-1 oder der GLP-1-Agonist Exendin-4, Exendin-3; oder ein Analogon oder Derivat davon ist.

14. Verwendung nach einem der Ansprüche 1 und 3 bis 13 oder GLP-1 oder GLP-1-Agonist nach einem der Ansprüche 2 bis 13, worin das Medikament mit zumindest einem Antidiabetes-Arzneimittel verabreicht wird.

15. Verwendung oder GLP-1 oder GLP-1-Agonist nach Anspruch 14, worin:
(a) die Verabreichung unter etwa einer therapeutisch wirksamen Menge für zumindest eines des GLP-1 oder GLP-1-Agonisten oder das Antidiabetes-Arzneimittel in dem Säugetier liegt; oder
(b) die Verabreichung zumindest etwa bei einer therapeutisch wirksamen Menge für zumindest eines des GLP-1 oder GLP-1-Agonisten oder das Antidiabetes-Arzneimittel in dem Säugetier liegt.

16. Verwendung nach einem der Ansprüche 1 und 3 bis 15 oder GLP-1 oder GLP-1-Agonist nach einem der Ansprüche 2 bis 15, worin die Verabreichung des Antidiabetes-Arzneimittels vor oder nach der arzneimittelfreien Zeitspanne erfolgt.

17. Verwendung nach einem der Ansprüche 1 und 3 bis 16 oder GLP-1 oder GLP-1-Agonist nach einem der Ansprüche 2 bis 16, worin zumindest eines der Antidiabetes-Arzneimittel Insulin, ein Insulin-Analogon oder ein pharmazeutisch annehmbares Gemisch davon ist.

18. Verwendung oder GLP-1 oder GLP-1-Agonist nach Anspruch 17, worin:
(a) das Insulin menschliches Insulin, Rinderinsulin, Schweineinsulin oder ein Gemisch daraus ist; oder
(b) das Insulin-Analogon menschliches Lys(B28),Pro(B29)-Insulin ist; oder
(c) das Antidiabetes-Arzneimittel ein Sulfonylharnstoff, Biguanid, Thiazolidindion, Diazoxid, Somatostatin oder ein α-Glucosidase-Inhibitor ist.

19. Verwendung oder GLP-1 oder GLP-1-Agonist nach Anspruch 18, worin:
(a) der Sulfonylharnstoff aus der aus Tolbutamid, Chlorpropamid, Tolazamid, Acetohexamid, Glyburid, Glipizid und Gliclazid bestehenden Gruppe ausgewählt ist; oder
(b) das Biguanid Metformin oder Phenformin ist; oder
(c) das Thiazolidindion Ciglitazon oder Pioglitazon ist; oder
(d) der α-Glucosidase-Inhibitor Acarbose ist.

20. Verwendung nach einem der Ansprüche 1 und 3 bis 19 oder GLP-1 oder GLP-1-Agonist nach einem der Ansprüche 2 bis 19, worin das Säugetier ein menschliches Individuum ist, das Diabetes mellitus oder eine verwandte Erkrankung aufweist oder bei dem dieser/eine solche vermutet wird.

21. Verwendung oder GLP-1 oder GLP-1-Agonist nach Anspruch 20, worin:
(a) der Diabetes mellitus aus der aus insulinabhängigem Diabetes mellitus (IDDM oder Typ-I-Diabetes) und nicht-insulinabhängigem Diabetes mellitus (NIDDM oder Typ-II-Diabetes) bestehenden Gruppe ausgewählt ist; oder
(b) das menschliche Individuum, bei dem Diabetes mellitus vermutet wird, eine genetische Prädisposition zur Entwicklung der Krankheit aufweist; oder
(c) die mit Diabetes mellitus verwandte Erkrankung aus der aus verminderter Glukose-Toleranz (IGT), sich während der Pubertät manifestierendem Diabetes mellitus (MODY); Leprechaunismus (Insulinrezeptor-Mutation), tropischem Diabetes, sekundärem Diabetes aufgrund einer Pankreaserkrankung oder einem diesbezüglichen chirurgischen Eingriff; Diabetes, der mit einem genetischen Syndrom assoziiert ist (z.B. Prader-Willi-Syndrom); Pankreatitis; und sekundärem Diabetes aufgrund von Endokrinopathien; Adipositas; sowie metabolischem Syndrom (Snydrom X) bestehenden Gruppe ausgewählt ist.

## Revendications

1. Utilisation d'au moins un GLP-1 ou d'un agoniste du GLP-1 ayant un effet de GLP-1 pour la fabrication d'un médicament pour utilisation dans une méthode de prévention ou de traitement du diabète dans un mammifère, où la méthode comprend la réduction de l'administration du GLP-1 ou de l'agoniste du GLP-1 en dessous d'environ la quantité thérapeutiquement efficace pour un temps favorable à la production d'une fenêtre thérapeutique, où la fenêtre thérapeutique est définie en outre comme un intervalle de temps entre un premier point limite suivant la réduction de l'administration du GLP-1 ou de l'agoniste du GLP-1 et un deuxième point limite.

2. GLP-1 ou agoniste du GLP-1 ayant un effet GLP-1, pour utilisation dans une méthode de traitement ou de prévention du diabète dans un mammifère, la méthode comprenant la réduction de l'administration du GLP-1 ou de l'agoniste du GLP-1 en dessous d'environ la quantité thérapeutiquement efficace durant un temps favorable à la production d'une fenêtre thérapeutique, où la fenêtre thérapeutique est en outre définie comme un intervalle de temps entre un premier point limite suivant la réduction de l'administration du GLP-1 ou de l'agoniste du GLP-1 et un deuxième point limite.

3. Utilisation selon la revendication 1 ou le GLP-1 ou agoniste du GLP-1 selon la revendication 2, dans laquelle:
(a) l'administration du GLP-1 ou de l'agoniste du GLP-1 est réduite durant la fenêtre thérapeutique selon au moins environ 50% en dessous de la quantité thérapeutique; ou
(b) l'administration du GLP-1 ou de l'agoniste du GLP-1 est réduite durant la fenêtre thérapeutique selon au moins environ 90% en dessous de la quantité thérapeutique.

4. Utilisation selon la revendication 1 ou la revendication 3, où le GLP-1 ou agoniste du GLP-1 selon la revendication 2 ou la revendication 3, où l'administration du GLP-1 ou de l'agoniste du GLP-1 est arrêtée durant la fenêtre thérapeutique.

5. Utilisation selon la revendication 1 ou le GLP-1 ou agoniste du GLP-1 selon la revendication 2, où le deuxième point limite est défini par un test FBG standard ou d'hémoglobine glycosylée.

6. Utilisation selon l'une quelconque des revendications 1 et 3 à 5 ou le GLP-1 ou agoniste du GLP-1 selon l'une quelconque des revendications 2 à 5, dans laquelle:
(a) la fenêtre thérapeutique est d'environ un jour à environ vingt-cinq semaines; ou bien
(b) la fenêtre thérapeutique est entre environ trois à quatre semaines.

7. Utilisation selon l'une quelconque des revendications 1 et 3 à 6 ou le GLP-1 ou agoniste du GLP-1 selon l'une quelconque des revendications 2 à 6, dans laquelle:
(a) le GLP-1 ou agoniste du GLP-1 est administré comme une formulation à effet retard; ou bien
(b) le GLP-1 ou agoniste du GLP-1 est administré au bol du mammifère au moins environ une fois par jour; ou bien
(c) le GLP-1 ou agoniste du GLP-1 est administré au bol du mammifère au moins une fois par semaine; ou bien
(d) l'administration du GLP-1 ou de l'agoniste du GLP-1 est d'environ deux fois par jour (i.v. ou subcutanée) pour une durée entre environ un à environ vingt semaines.

8. Utilisation selon l'une quelconque des revendications 1 et 3 à 7 ou le GLP-1 ou agoniste du GLP-1 selon l'une quelconque des revendications 2 à 7, la méthode comprenant en outre l'administration au mammifère d'une deuxième quantité thérapeutiquement efficace du GLP-1 ou de l'agoniste du GLP-1 à la suite de la fenêtre thérapeutique.

9. Utilisation ou le GLP-1 ou agoniste du GLP-1 selon la revendication 8, la méthode comprenant en outre la réduction de l'administration de la deuxième quantité thérapeutiquement efficace du GLP-1 ou de l'agoniste du GLP-1 durant un temps favorable à la production d'une deuxième fenêtre thérapeutique.

10. Utilisation selon l'une quelconque des revendications 1 et 3 à 9 ou le GLP-1 ou l'agoniste du GLP-1 selon l'une quelconque des revendications 2 à 9, dans laquelle dans la méthode:
(a) les étapes d'administration et de réduction sont répétées au moins une fois; ou bien
(b) les étapes d'administration et de réduction sont répétées au moins environ 2 à environ 25 fois; ou bien
(c) les étapes d'administration et de réduction sont répétées selon le besoin pour prévenir ou traiter le diabète ou un trouble associé.

11. Utilisation du GLP-1 ou de l'agoniste du GLP-1 selon la revendication 10, dans laquelle la méthode est pratiquée sur la durée de vie du mammifère.

12. Utilisation selon l'une quelconque des revendications 1 et 3 à 11 ou le GLP-1 ou l'agoniste du GLP-1 selon l'une quelconque des revendications 2 à 11, où le GLP-1 ou l'agoniste du GLP-1 est administré au mammifère à une dose d'au moins environ 0,01 nmol/kg (poids corporel).

13. Utilisation selon l'une quelconque des revendications 1 et 3 à 12 ou le GLP-1 ou l'agoniste du GLP-1 selon l'une quelconque des revendications 2 à 12, où le GLP-1 ou l'agoniste du GLP-1 est l'exendine-4, l'exendine-3; ou un analogue ou dérivé de celle-ci.

14. Utilisation selon l'une quelconque des revendications 1 et 3 à 13 ou le GLP-1 ou agoniste du GLP-1 selon l'une quelconque des revendications 2 à 13, dans laquelle le médicament est administré avec au moins un médicament anti-diabétique.

15. Utilisation ou le GLP-1 ou agoniste du GLP-1 selon la revendication 14, dans laquelle:
(a) l'administration est en dessous d'environ une quantité thérapeutiquement efficace pour au moins un parmi le GLP-1 ou l'agoniste du GLP-1, ou bien le médicament anti-diabétique dans le mammifère; ou bien
(b) l'administration est au moins environ à une quantité thérapeutiquement efficace pour au moins un parmi le GLP-1 ou l'agoniste du GLP-1, ou bien le médicament anti-diabétique dans le mammifère.

16. Utilisation selon l'une quelconque des revendications 1 et 3 à 15 ou le GLP-1 ou agoniste du GLP-1 selon l'une quelconque des revendications 2 à 15, dans laquelle l'administration du médicament anti-diabétique a lieu avant ou après la fenêtre thérapeutique.

17. Utilisation selon l'une quelconque des revendications 1 et 3 à 16 ou le GLP-1 ou agoniste du GLP-1 selon l'une quelconque des revendications 2 à 16, dans laquelle au moins un des médicaments anti-diabétiques est l'insuline, un analogue de l'insuline ou un mélange pharmaceutiquement acceptable de ceux-ci.

18. Utilisation ou le GLP-1 ou agoniste du GLP-1 selon la revendication 17, dans laquelle:
(a) l'insuline est de l'insuline humaine, l'insuline bovine, l'insuline porcine; ou un mélange de celles-ci; ou
(b) l'analogue de l'insuline est l'insuline humaine Lys(B28), Pro(B29); ou
(c) le médicament anti-diabétique est une sulfonylurée, biguanide, thiazolidinedione, diazoxyde, somatostatine ou un inhibiteur d'alpha-glucosidase.

19. Utilisation ou le GLP-1 ou agoniste du GLP-1 selon la revendication 18, dans laquelle:
(a) la sulfonylurée est sélectionnée dans le groupe consistant en tolbutamide, chlorpropamide, tolazamide, acétohexamide, glyburide, glipizide et glicazide; ou
(b) la biguanide est metformine ou phenformine; ou
(c) la thiazolidinedione est ciglitazone ou pioglitazone; ou
(d) l'inhiteur d'alpha-glucosidase est l'acarbose.

20. Utilisation selon l'une quelconque des revendications 1 et 3 à 19 ou le GLP-1 ou agoniste du GLP-1 selon l'une quelconque des revendications 2 à 19, dans laquelle le mammifère est un sujet humain qui a ou dont on pense qu'il a du diabète sucré ou un trouble associé.

21. Utilisation ou le GLP-1 ou agoniste du GLP-1 selon la revendication 20, dans laquelle:
(a) le diabète sucré est sélectionné dans le groupe consistant en diabète sucré insulino-dépendant (IDDM ou diabète de type I) et diabète sucré non-insulino-dépendant (NIDDM, ou diabète de type II); ou
(b) le sujet humain dont on pense qu'il a le diabète sucré est génétiquement prédisposé pour développer la maladie; ou
(c) le trouble lié au diabète sucré est sélectionné dans le groupe consistant en tolérance réduite au glucose (IGT), diabète juvénil de l'adulte (MODY); lépréchaunisme (mutation du récepteur de l'insuline), diabète tropical, diabète secondaire à une maladie ou chirurgie du pancréas; diabète associé à un syndrome génétique (par exemple, syndrome Prader-Willi); pancréatite; et diabète secondaire à des endocrinopathies; adipositas; et syndrome métabolique (syndrome X).
